# EUROPEAN PATENT SPECIFICATION

(11) **EP 3 691 725 B1**
(45) Date of publication and mention of the grant of the patent: **09.04.2025**
(21) Application number: 18864017.1
(22) Date of filing: 05.10.2018
(51) Int. Cl.: A61M 16/10, A61M 16/12, A61M 16/16, A63B 23/18, A63B 24/00, A61M 16/00, A61M 16/04, A61M 16/06

(54) **SYSTEMS AND METHODS FOR HYPOXIC GAS DELIVERY FOR ALTITUDE TRAINING AND ATHLETIC CONDITIONING**
SYSTEME UND VERFAHREN ZUR HYPOXISCHEN GASVERSORGUNG FÜR HÖHENTRAINING UND ATHLETISCHE KONDITIONIERUNG
SYSTÈMES ET PROCÉDÉS DE DISTRIBUTION DE GAZ HYPOXIQUE POUR L'ENTRAÎNEMENT EN ALTITUDE ET LE CONDITIONNEMENT ATHLÉTIQUE

(30) Priority: 06.10.2017 US 201762569147 P
(43) Date of publication of application: 12.08.2020
(73) Proprietor: Fisher & Paykel Healthcare Limited, East Tamaki, Auckland 2013 (NZ)
(72) Inventor: O'DONNELL, Kevin Peter, Auckland 2013 (NZ); KAMOLINS, Christopher Charles, Auckland 2013 (NZ); KIRTON, Robert Stuart, Auckland 2013 (NZ)
(74) Representative: Dehns
(86) International application number: PCT/NZ2018/050136
(87) International publication number: WO 2019/070135

(56) References cited:
- WO-A1-2016/133406
- US-A1- 2005 247 311
- US-A1- 2006 185 669
- US-A1- 2006 196 502
- US-A1- 2007 077 200
- US-A1- 2008 078 385
- US-A1- 2009 183 738
- US-A1- 2010 282 257
- US-A1- 2016 095 994

## Description

### FIELD OF THE DISCLOSURE

The present disclosure relates in some aspects to systems and methods for high flow hypoxic gas delivery to a user via a nasal conduit. The gas delivery can be for a wide variety of indications, including but not limited to altitude training and improving cardiovascular conditioning and athletic performance.

### BACKGROUND

Altitude effects can influence athletic performance. Some athletes have found it advantageous to spend time, such as live and/or train, at relatively high altitudes to gain a competitive edge, commonly known as altitude training. Not to be limited by theory, altitude training has several beneficial potential physiological effects. At high altitude the partial pressure of oxygen is reduced due to the reduced barometric pressure, even though the actual concentration of oxygen remains relatively constant. Over the course of multiple weeks, a person's body may adapt to this relatively low oxygen environment by increasing mass of red blood cells and hemoglobin, and/or by altering muscle metabolism. These physiological changes can be shown to have follow up positive effects, particularly in terms of a raised VO₂ max, which is the maximum rate at which oxygen can be used be consumed by the body. VO₂ max is strongly linked to aerobic capacity, and in turn has effects on speed, strength, endurance and recovery.

There can be a large number of variables associated with altitude training, not limited to types of exercise, levels of altitude/oxygen concentration, length of therapy sessions, total length of therapy regime, length of time since leaving altitude, whether or not to remain at altitude for training sessions, whether or not to remain at altitude outside of training sessions, efficacy of simulated altitude, optimum ways to deliver simulated altitude, whether to control to inspired oxygen or blood oxygen saturation, as well as determining the exact underlying mechanism behind the physiological changes. As actually being at altitude poses multiple challenges in terms of cost, inconvenience and difficulty to alter the exact altitude as noted above, methods of generating an artificial high altitude environment have been investigated. As one theory for the effects of altitude training is the reduced partial pressure of oxygen, this could be accurately mimicked by adding one, two, or more hypoxic gases, such as nitrogen or other gases to ambient air, resulting in a gas with a reduced oxygen concentration. Generating a hypoxic environment can be shown to artificially replicate the effects of being at altitude. Systems and methods that can more efficiently, effectively, safely, and conveniently replicate such altitude and other effects are needed.

WO2016/133406A1 discloses a system and a method for oxygenating a patient in relation to anesthesia using high flow gas delivery, wherein the oxygenation requirements of the patient are determined before or during anesthesia. US2006/185669A1 discloses an apparatus for the delivery of hypoxic air to a user comprising a biofeedback means of control wherein at least one physiologically measurable parameter of the user is constantly measured. US 2007/077200 A1 discloses a system, device, and method for automatically inducing, maintaining, or controlling hypoxia in a patient, wherein at least one physiological parameter of the patient is monitored and the delivery of the hypoxic gas mixture is automatically controlled based on a value of the physiological parameter.

### SUMMARY OF THE INVENTION

The present invention is directed to a nasal high flow system according to claim 1. Additional features and embodiments of the invention are defined in the dependent claims.

SUMMARY OF THE DISCLOSURE In a first aspect, there is provided a nasal high flow system configured for providing a hypoxic flow of gases to a user includes an apparatus comprising a gas flow path, an ambient air inlet, and a hypoxic gas source inlet configured to connect to a hypoxic gas source and configured to create a hypoxic gas composition upon mixing of ambient air and the hypoxic gas; a user interface comprising a nasal cannula comprising nasal prongs; a gas flow generation element configured to deliver the hypoxic gas composition to nares of the user at a predetermined high flow rate of at least about 10 liters/minute; a sensor configured to measure a physiological parameter of the user, wherein the physiological parameter is blood oxygen saturation; and a controller configured to automatically control the delivery of the hypoxic gas composition based on the measured physiological parameter; wherein the nasal cannula is non-sealed.

In some configurations, the gas flow generation element can comprise one or more of a blower, a compressor, a pressurized tank, or a gas source in the wall.

In some configurations, the system can further include the hypoxic gas source.

In some configurations, the hypoxic gas source can comprise a hypoxic gas reservoir.

In some configurations, the hypoxic gas can comprise enriched nitrogen.

In some configurations, the gas flow generation element can be configured to deliver the hypoxic gas composition sufficient to meet the peak inspiratory demand of the user.

In some configurations, the system can further include a heating element configured to heat the hypoxic gas composition prior to reaching the nares of the user. In some configurations, the heating element can comprise a heater plate. In some configurations, the heating element can comprise a heater wire.

In some configurations, the system can further include a humidification element configured to humidify the hypoxic gas composition prior to reaching the nares of the user. The humidification element may be a humidification chamber.

In some configurations, the humidification element can be configured to humidify the hypoxic gas composition to a relative humidity of at least about 80%. The humidification element can be configured to humidify the hypoxic gas composition to a relative humidity of at least about 90%. The humidification element can be configured to humidify the hypoxic gas composition to a relative humidity of at least about 95%. The humidification element can be configured to humidify the hypoxic gas composition to a relative humidity of at least about 99%. The humidification element can be configured to humidify the hypoxic gas composition to a relative humidity of at least about 100%.

In some configurations, the system can further include a sensor configured to measure a peak inspiratory demand of the user; and the system can be configured to adjust the flow rate based upon the measured peak inspiratory demand.

In some configurations, the system can be configured to deliver the hypoxic gas in an oxygen concentration of between about 10% and about 20%. The system can be configured to deliver the hypoxic gas in an oxygen concentration of between about 15% and about 20%. The system can be configured to deliver the hypoxic gas in an oxygen concentration of between about 10.2% and about 20.9%. The system can be configured to deliver the hypoxic gas in an oxygen concentration of between about 11.9% and about 17.4%. The system can be configured to deliver the hypoxic gas in an oxygen concentration of between about 13.8% and about 17.4%. The system can be configured to deliver the hypoxic gas in an oxygen concentration of between about 15.7% and about 16.7%.

In some configurations, the controller can be configured to automatically control the delivery of the hypoxic gas composition by adjusting an amount of hypoxic gas mixed into the hypoxic gas composition. In some configurations, the controller can be configured to automatically controlling the delivery of the hypoxic gas composition by adjusting an amount of enriched nitrogen mixed into the hypoxic gas composition.

In some configurations, the physiological parameter can further comprise at least one selected from the group consisting of: heart rate, respiratory rate, heart rate variability, and blood pressure.

In some configurations, the system can further include an alarm configured to notify the user when the physiological parameter deviates from a pre-selected range for at least a pre-determined period of time.

In some configurations, the alarm can comprise one of a visual, auditory, or tactile alarm.

In some configurations, the controller can be configured to titrate the composition of the hypoxic gas composition to achieve a blood oxygen saturation of the user of less than about 95%. The controller can be configured to titrate the composition of the hypoxic gas composition to achieve a blood oxygen saturation of the user of between about 80% and about 94%. The controller can be configured to titrate the composition of the hypoxic gas composition to achieve a blood oxygen saturation of the user of between about 85% and about 92%. The controller can be configured to titrate the composition of the hypoxic gas composition to achieve a blood oxygen saturation of the user of between about 87% and about 90%. The controller can be configured to titrate the composition of the hypoxic gas composition to achieve a blood oxygen saturation of the user of between about 80% and about 85%.

In some configurations, the system can further include a sensor configured to sense the oxygen concentration of the hypoxic gas composition.

In some configurations, the system can further include a display, wherein the system is configured to output the sensed oxygen concentration in real time to the display.

In some configurations, the system can be further configured to correlate the sensed oxygen concentration to an altitude equivalent using a processor, and outputting the altitude equivalent in real time to the display.

In some configurations, the system can be further configured to obtain a target oxygen concentration of the hypoxic gas composition based on the blood oxygen saturation.

In some configurations, the system can be further configured to achieve the target oxygen concentration based on the sensed oxygen concentration.

In some configurations, the controller can be configured to achieve the target oxygen concentration by controlling a hypoxic gas inlet valve.

In some configurations, the controller can be configured to alter a flow rate of the hypoxic gas into the hypoxic gas composition.

In some configurations, the gas flow generation element can be configured to deliver the hypoxic gas composition to the nares of the user at a predetermined flow rate of at least about 20 liters/minute. The gas flow generation element can be configured to deliver the hypoxic gas composition to the nares of the user at a predetermined flow rate of at least about 30 liters/minute. The gas flow generation element can be configured to deliver the hypoxic gas composition to the nares of the user at a predetermined flow rate of at least about 40 liters/minute. The gas flow generation element can be configured to deliver the hypoxic gas composition to the nares of the user at a predetermined flow rate of at least about 50 liters/minute. The gas flow generation element can be configured to deliver the hypoxic gas composition to the nares of the user at a predetermined flow rate of at least about 60 liters/minute. The gas flow generation element can be configured to deliver the hypoxic gas composition to the nares of the user at a predetermined flow rate of at least about 70 liters/minute.

In some configurations, the nasal high flow system can be a high flow device.

In some configurations, the high flow device can be portable.

In some configurations, the hypoxic gas composition can comprise at least one physiologically inert gas. In some configurations, the hypoxic gas composition can comprise nitrogen.

In some configurations, heating the hypoxic gas composition can be sufficient to inactivate a pathogen of interest.

In some configurations, the pathogen of interest can be a human rhinovirus or influenza.

In some configurations, humidifying and heating the hypoxic gas composition can comprise humidifying and heating the hypoxic gas composition to a dew point temperature of between about 37°C and about 45°C. Humidifying and heating the hypoxic gas composition can comprise humidifying and heating the hypoxic gas composition to a dew point temperature of between about 37°C and about 43°C. Humidifying and heating the hypoxic gas composition can comprise humidifying and heating the hypoxic gas composition to a dew point temperature of between about 43°C and about 45°C. Humidifying and heating the hypoxic gas composition can comprise humidifying and heating the hypoxic gas composition to a dew point temperature of between about 40°C and about 43°C. Humidifying and heating the hypoxic gas composition can comprise humidifying and heating the hypoxic gas composition to a dew point temperature of about 41°C.

In some configurations, humidifying the hypoxic gas composition can comprise humidifying the hypoxic gas composition to a relative humidity of at least about 80%. Humidifying the hypoxic gas composition can comprise humidifying the hypoxic gas composition to a relative humidity of at least about 90%. Humidifying the hypoxic gas composition can comprise humidifying the hypoxic gas composition to a relative humidity of at least about 95%. Humidifying the hypoxic gas composition can comprise humidifying the hypoxic gas composition to a relative humidity of at least about 99%. Humidifying the hypoxic gas composition can comprise humidifying the hypoxic gas composition to a relative humidity of at least about 100%.

In some configurations, an oxygen concentration of the hypoxic gas composition can be between about 10% and about 20%. An oxygen concentration of the hypoxic gas composition can be between about 15% and about 20%. An oxygen concentration of the hypoxic gas composition can be between about 10.2% and about 20.9%. An oxygen concentration of the hypoxic gas composition can be between about 11.9% and about 17.4%. An oxygen concentration of the hypoxic gas composition can be between about 13.8% and about 17.4%. An oxygen concentration of the hypoxic gas composition can be between about 15.7% and about 16.7%.

In some configurations, the measured physiological parameter can further comprise at least one selected from the group consisting of: heart rate, respiratory rate, heart rate variability, and blood pressure.

In some configurations, automatically controlling the delivery of the hypoxic gas composition can comprise adjusting an amount of hypoxic gas mixed into the hypoxic gas composition.

In some configurations, notifying the user can comprise activating a visual, auditory, or tactile alarm.

In some configurations, automatically controlling the delivery of the hypoxic gas can comprise titrating the composition of the hypoxic gas composition to achieve a blood oxygen saturation of less than about 95%. Automatically controlling the delivery of the hypoxic gas can comprise titrating the composition of the hypoxic gas composition to achieve a blood oxygen saturation of between about 80% and about 94%. Automatically controlling the delivery of the hypoxic gas can comprise titrating the composition of the hypoxic gas composition to achieve a blood oxygen saturation of between about 85% and about 92%. Automatically controlling the delivery of the hypoxic gas can comprise titrating the composition of the hypoxic gas composition to achieve a blood oxygen saturation of between about 87% and about 90%. Automatically controlling the delivery of the hypoxic gas can comprise titrating the composition of the hypoxic gas composition to achieve a blood oxygen saturation of between about 80% and about 85%.

In some configurations, titrating the composition of the hypoxic gas composition can comprise altering a flow rate of nitrogen into the hypoxic gas composition.

In some configurations, the hypoxic gas composition can be delivered to the user at a flow rate of at least about 20 liters/minute. The hypoxic gas composition can be delivered to the user at a flow rate of at least about 30 liters/minute. The hypoxic gas composition can be delivered to the user at a flow rate of at least about 40 liters/minute. The hypoxic gas composition can be delivered to the user at a flow rate of at least about 50 liters/minute. The hypoxic gas composition can be delivered to the user at a flow rate of at least about 60 liters/minute. The hypoxic gas composition can be delivered to the user at a flow rate of at least about 70 liters/minute.

In some configurations, the hypoxic gas source can comprise a reservoir.

In some configurations, the hypoxic gas comprises a physiologically inert gas. The hypoxic gas can comprise enriched nitrogen.

In some configurations, the heating element and the humidification element can be configured to heat and humidify the hypoxic gas composition to a dew point temperature of between about 37°C and about 43°C. The heating element and the humidification element can be configured to heat and humidify the hypoxic gas composition to a dew point temperature of between about 37°C and about 43°C. The heating element and the humidification element can be configured to heat and humidify the hypoxic gas composition to a dew point temperature of between about 43°C and about 45°C. The heating element and the humidification element can be configured to heat and humidify the hypoxic gas composition to a dew point temperature of between about 40°C and about 43°C. The heating element and the humidification element can be configured to heat and humidify the hypoxic gas composition to a dew point temperature of about 41°C.

In some configurations, the system can further include a sensor configured to measure a physiological parameter of the user; and a controller configured to automatically control the delivery of the hypoxic gas composition based on the measured physiological parameter.

In some configurations, the system can be further configured to achieve the target oxygen concentration by controlling a hypoxic gas inlet valve.

In some configurations, delivering the hypoxic gas can be sufficient to meet peak inspiratory demand of the user.

In some configurations, heating the hypoxic gas composition can comprise heating the hypoxic gas composition to a temperature of between about 30°C and about 45°C. Heating the hypoxic gas composition can comprise heating the hypoxic gas composition to between about 37°C and about 43°C. Heating the hypoxic gas composition can comprise heating the hypoxic gas composition to about 41°C.

In some configurations, the user interface can comprise a mask.

In some configurations, the heating element can be configured to heat the hypoxic gas composition to a temperature of between about 30°C and about 45°C. The heating element can be configured to heat the hypoxic gas composition to between about 30°C and about 43°C. The heating element can be configured to heat the hypoxic gas composition to between about 37°C and about 43°C. The heating element can be configured to heat the hypoxic gas composition to about 41°C.

In some configurations, the heating element and the humidification element can be configured to heat and humidify the hypoxic gas composition to a dew point temperature of between about 30°C and about 45°C. The heating element and the humidification element can be configured to heat and humidify the hypoxic gas composition to a dew point temperature of between about 30°C and about 43°C. The heating element and the humidification element can be configured to heat and humidify the hypoxic gas composition to a dew point temperature of between about 37°C and about 43°C. The heating element and the humidification element can be configured to heat and humidify the hypoxic gas composition to a dew point temperature of about 41°C.

In some configurations, providing can comprise simulating a first altitude.

In some configurations, the system can include a screen configured to display an altitude equivalent of the altitude or fitness training.

In some configurations, the system can include a screen configured to display an SpO₂ of the user.

In some configurations, the screen can comprise a touchscreen.

In some configurations, the system can include user input devices for the user to select an altitude equivalent of the altitude or fitness training.

In some configurations, the altitude equivalent can be based on an oxygen concentration.

In some configurations, the oxygen concentration can be an FdO₂ set point or a sensed oxygen concentration.

In some configurations, the user input devices can comprise one or more buttons.

In some configurations, the user input devices can comprise a touchscreen.

In some configurations, the controller can be configured to vary the oxygen concentration of the hypoxic gas composition gradually. The controller can be configured to vary the oxygen concentration of the hypoxic gas composition in a series of step changes. The controller can be configured to vary the oxygen concentration of the hypoxic gas composition in a step change.

In some configurations, the controller can be configured to provide the hypoxic gas composition to simulate a first altitude.

In some configurations, the controller can be configured to vary the oxygen concentration of the hypoxic gas composition to simulate a second altitude lower than the first altitude.

In some configurations, the controller can be configured to provide a gas composition substantially the same as ambient air prior to providing the hypoxic gas composition.

In some configurations, the controller can be configured to vary the oxygen concentration of the hypoxic gas composition responsive to a change in the user's SpO₂.

In some configurations, they system can comprise a sensor configured to measure the user's SpO₂.

In some configurations, the system can comprise a humidification chamber.

In some configurations, the system can comprise a heating element.

In some configurations, the controller can be configured to automatically control the delivery of the hypoxic gas composition by adjusting an amount of hypoxic gas mixed into the hypoxic gas composition. In some configurations, the system further comprises a sensor configured to sense the oxygen concentration of the hypoxic gas composition. In some configurations, the system can be further configured to obtain a target oxygen concentration of the hypoxic gas composition based on the blood oxygen saturation.

### BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.
Figure 1 shows in diagrammatic form a hypoxic gas composition delivery apparatus in the form of a flow therapy apparatus.
Figure 2 is a front view of the flow therapy apparatus with a humidifier chamber in position and a raised handle/lever.
Figure 3 is a top view corresponding to Figure 2.
Figure 4 is a right side view corresponding to Figure 2.
Figure 5 is a left side view corresponding to Figure 2.
Figure 6 is a rear view corresponding to Figure 2.
Figure 7 is a front left perspective view corresponding to Figure 2.
Figure 8 is a front right perspective view corresponding to Figure 2.
Figure 9 is a bottom view corresponding to Figure 2.
Figure 10 shows a first configuration of an air and nitrogen inlet arrangement of the flow therapy apparatus.
Figure 11 shows a second configuration of an air and nitrogen inlet arrangement of the flow therapy apparatus.
Figure 12 is a transverse sectional view showing further detail of the air and nitrogen inlet arrangement of Figure 11.
Figure 13 is another transverse sectional view showing further detail of the air and nitrogen inlet arrangement of Figure 11.
Figure 14 is a longitudinal sectional view showing further detail of the air and nitrogen inlet arrangement of Figure 11.
Figure 15 is an exploded view of upper and lower chassis components of a main housing of the flow therapy apparatus.
Figure 16 is a front left side perspective view of the lower chassis of the main housing showing a housing for receipt of a motor/sensor module sub-assembly.
Figure 17A is a first underside perspective view of the main housing of the flow therapy apparatus showing a recess inside the housing for the motor/sensor module sub-assembly.
Figure 17B is a second underside perspective view of the main housing of the flow therapy apparatus showing the recess for the motor/sensor module sub-assembly.
Figures 18A-E illustrate various views of an example flow therapy apparatus.
Figure 19A illustrates a block diagram of a control system interacting with and/or providing control and direction to components of a respiratory assistance system.
Figure 19B illustrates a block diagram of an example controller.
Figure 20 illustrates a block diagram of a motor/sensor module.
Figure 21 illustrates a sensing chamber of an example removable motor/sensor module.
Figure 22 illustrates example block diagrams of a closed loop control system.

### DETAILED DESCRIPTION

Although certain examples are described below, those of skill in the art will appreciate that the present disclosure extends beyond the specifically disclosed examples and/or uses and obvious modifications and equivalents thereof. Thus, it is intended that the scope of protection of the present invention herein disclosed should not be limited by any particular examples described below but rather limited by the scope of the appended claims.

In some configurations, systems and methods can reduce the oxygen content of inspired air as a mechanism of simulating being at a higher altitude, improving athletic performance, and other medical and/or wellness benefits. One objective of this is to stimulate the body to produce more red blood cells, and thus oxygen carrying capacity as a way of increasing cardiovascular endurance. In some cases, the systems and methods can be used for a time period sufficient to stimulate erythropoiesis such that the user's hemoglobin level increases by about or at least about 5%, 10%, 15%, 20%, or more compared with prior to initiation of therapy, or ranges including any two of the aforementioned values.

Conventional devices that provide a hypoxic atmosphere within an enclosure can suffer from several disadvantages. Such conventional setups can be quite costly due to the volume of gas that needs to be altered. Many systems are not portable, and the ones that are (such as a tent) can be both difficult to transport as well as quite small, limiting what a user can do while using it. The user can also be prevented/restricted from leaving the enclosure, and others who do not wish to use the device are unable to enter it, thus limiting the ability for interaction between participants and non-participants, especially during extended use. If the user needs to suddenly end the therapy they need to leave the enclosure, which would take significantly longer than simply removing a patient interface, such as a cannula (some devices provide a breathing device with normoxic air for use in an emergency, but this can just further add to the cost). The use of a room would also prevent individually adjusted therapy when used with others, as everyone would be required to breathe the same gas composition. The volume of the room would also make it difficult to make any quick changes to the composition of the gas. As such, it would be advantageous to limit the hypoxic atmosphere to a user interface proximate to, or directly attachable to a user.

Devices that use a mask can be disadvantageous in some cases by their obstructive nature. Using a nasal cannula (e.g., as the only conduit to the user) instead of and without using a mask means the user can still talk, eat and drink while the device is attached, making it more viable for longer therapy sessions, as the user would not need to remove it for such tasks. The portability of the cannula can also allow the user to move the device with them as they do various activities throughout the day. These factors combined allow such devices to advantageously implement a wide range of therapy regimes. The user could have a nasal cannula attached and connected to high flow systems as disclosed herein during a majority of day to day tasks, while sleeping, and while exercising on a spot (such as with a treadmill or weight training). The cannula could also be removed and reattached fairly easily, which could be useful if the therapy regime required regularly stopping and starting the administration of the hypoxic gases. The cannula can also be far more comfortable, and does not run the risk of the user developing pressure sores. Users may also in some cases find a mask somewhat claustrophobic due to the amount of their face it covers. A cannula can also be safer than a mask in some cases, as it would not obstruct breathing if the device were to fail, and can be removed more easily if needed.

Examples of systems and methods as disclosed herein advantageously deliver high flow rates (e.g., greater than about 10 liters per minute in adults and/or in excess of peak inspiratory demand in some cases). Some conventional devices deliver a small dose of pure nitrogen upon inspiration that can mix with additional inspired air through the user's mouth and/or around the cannula to create the required hypoxic mixture. This method can have several disadvantages in some cases. One disadvantage is that as conventional devices deliver a fixed dose or low flow of nitrogen, the total fraction of inspired oxygen (FiO₂) would change as the inspiratory flow rate changes, and would depend on the user's inspiratory tidal volume, meaning that a change in breath rate or volume would alter the composition being delivered. This could make targeting a specific FiO₂ or SpO₂ very challenging. In contrast, systems and methods as disclosed herein can deliver flow rates in excess of the user's inspiratory demand, which can advantageously provide a more consistent and controlled hypoxic composition regardless of changes in the user's breathing. At least two parameters can be used to determine oxygen delivery to the user, FiO₂ and FdO₂. FiO₂ is the fraction of inspired oxygen, which is the oxygen concentration of the gas that the user is actually breathing in. FdO₂ is the fraction of delivered oxygen, which is the oxygen concentration of the gas flowing out of the cannula. FdO₂ can be directly measured by the flow therapy system, such as by measuring the oxygen concentration of the gas flowing through the system. FiO₂ is dependent on FdO₂ as well as the proportion of ambient air, if any, that is entrained when the user breathes in. While FiO₂ can be a preferred parameter in some cases in assessing the effect of the gas on the user, control of FdO₂ can allow the target FiO₂ be achieved, for example, due to the assumption that those two parameters have the same or substantially the same value in a high flow therapy system. During a high flow therapy session, the oxygen concentration measured in the system, FdO₂, can be substantially the same as the oxygen concentration the user is breathing, FiO₂, when the flow rate of gas delivered meets or exceeds the peak inspiratory demand of the user. That is, the volume of gas delivered by the system to the user during inspiration meets, or is in excess of, the volume of gas inspired by the user during inspiration. In that situation, no ambient air is entrained (e.g., around a non-sealed nasal cannula) and hence the value of FdO₂ is equal to the value of FiO₂. Accordingly, high flow therapies can help to prevent entrainment of ambient air when the user breathes in, as well as to flush the user's airways of expired gas. Further, systems and methods as disclosed herein can also provide a closed loop control to provide hypoxic composition, such as based on monitoring of the user's oxygen saturation, SpO₂, when the flow rate is not meeting the user's inspiratory demand such that FdO₂ is not equal to FiO₂ due to entrainment of ambient air.

While nitrogen is a common hypoxic gas that can be utilized in creating a hypoxic gas mixture, the use of other hypoxic gases are possible in addition to, or instead of nitrogen. In some examples, the hypoxic gas composition can include, or consist entirely of or substantially entirely of physiologically inert gases. Some examples include, but are not limited to nitrogen, heliox, nitric oxide, carbon dioxide, argon, helium, methane, sulfur hexafluoride, and combinations thereof.

In some cases, systems and methods as disclosed herein can advantageously not require or perform any detection of when the user breathes in, as a fixed flow rate of gas can be delivered at all times. Systems and methods can also provide a safe, predictable, precise, hypoxic gas composition that avoids the risk of pooling large quantities of nitrogen, such as in situations where the user temporarily breathes through their mouth or the breath synchronization is not used.

Conventional systems can also suffer from the disadvantage of at best only being able to control the composition of gas up to the user's face. The actual composition of gas in the airways of the user would vary based on respiratory volume, respiratory rate, gas exchange rates of the lung, among other factors. Due to the high flow of gas being delivered by some examples as disclosed herein, the user's airways can be constantly flushed of old expired gas, so when the user inspires, the gas in user's upper airways would match that being delivered from the device or system, regardless of the aforementioned factors. Nasal high-flow gas delivery systems have been disclosed to enhance oxygen delivery to patients, but not to provide the opposite effect of delivering a hypoxic gas composition for various indications.

In some configurations, the use of a nasal system (with or without high flow delivery) to deliver warm and/or humidified hypoxic gas compositions could be beneficial in recovery from physical activity. One current criticism of altitude training is that the hypoxic conditions lead to lower intensity during training and poorer recovery after training, and that these factors have the potential to counteract out the benefits of the body's acclimatization to altitude. By combining the hot and/or humidified gas delivery of the nasal high flow system with an altitude training program, a user could potentially gain the benefits of natural acclimatization with reduced negative side effects that would otherwise impede performance and reduce overall benefits. In some cases, heated and/or humidified air can prevent or treat infections by various pathogens as discussed elsewhere herein, including via raising the temperature, or creating enough heat energy in the nasal passage, to kill some or all of the pathogens.

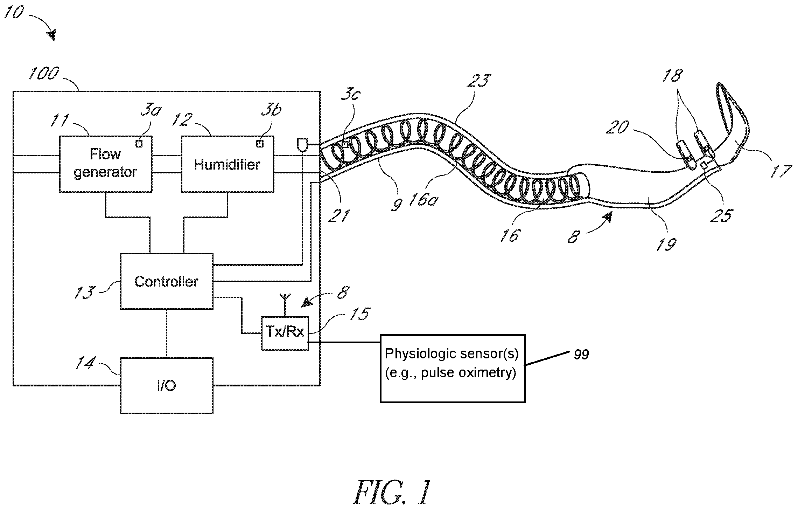

A schematic representation of a respiratory system or flow therapy apparatus 10 is provided in Figure 1. The apparatus 10 can include a main housing 100. The main housing 100 can contain a flow generator 11 that can be in the form of a motor/impeller arrangement, an optional humidifier or humidification chamber 12, a controller 13, and a user interface 14. The apparatus 10 can include any suitable gas flow generation element. The gas flow generation element can be used to generate a flow of gas and can include one or more flow generators and/or sources of pressurized gas. Examples of flow generators can include a blower or a compressor. Examples of sources of pressurized gas can include a pressurized tank or a gas source in the wall. The user interface 14 can include a display and input device(s) such as button(s), a touch screen, a combination of a touch screen and button(s), or the like. The controller 13 can include a hardware processor and can be configured or programmed to control the components of the apparatus, including but not limited to operating the flow generator 11 to create a flow of gases (e.g., hypoxic gases) for delivery to a user, operating the humidifier 12 (if present) to humidify and/or heat the gases flow, receiving user input from the user interface 14 for reconfiguration and/or user-defined operation of the apparatus 10, and outputting information (for example on the display) to the user or healthcare professional or anyone else viewing the apparatus.

With continued reference to Figure 1, a user breathing conduit 16 can be coupled to a gases flow outlet 21 in the housing 100 of the flow therapy apparatus 10, and be coupled to a user interface 17, which can be a non-sealing interface such as a nasal cannula with a manifold 19 and nasal prongs 18. The term "non-sealing interface" as used herein can refer to an interface providing a pneumatic link between an airway of a user and a gases flow source (such as from flow generator 11) that does not completely occlude the airway of the user. A non-sealed pneumatic link can comprise an occlusion of less than about 95*%* of the airway of the user. The non-sealed pneumatic link can comprise an occlusion of less than about 90*%* of the airway of the user. The non-sealed pneumatic link can comprise an occlusion of between about 40*%* and about 80*%* of the airway of the user. The airway can include one or more of a nare or mouth of the user. Additionally, or alternatively, the user breathing conduit 16 can be coupled to a face mask, or a tracheostomy interface. The gases flow that is generated by the flow therapy apparatus 10, and which may be humidified, is delivered to the user via the inspiratory conduit 16 through the cannula 17. The inspiratory conduit 16 can have a heater wire 16a to heat gases flow passing through to the user. The heater wire 16a can be under the control of the controller 13. In some embodiments, the device could include a gas heating mode in which the heater wire 16a or another heating element can be utilized to heat the hypoxic gases above the temperature of gases in the user's airway, and/or provide heat energy sufficient to kill or otherwise inactivate pathogens (e.g. viruses, including human rhinoviruses, influenza, and the like; fungi; and/or bacteria) from the gases flow, and/or pathogens already in the airways or elsewhere within the user. In some embodiments, a heating element can be present within or otherwise associated with a humidifier to raise the actual temperature and/or dew point temperature of the humidified gases stream to create a heated, humidified hypoxic gas composition, as discussed further below. Killing pathogens as both a therapeutic and/or preventative measure could be beneficial especially to athletes, where illness could severely undermine any training regime or competition performance. In some configurations, the gas heating mode can raise the temperature in the user's nasal passage high enough to provide heat energy for killing some or all of the pathogens, but not to the extent that the gases are too hot and cause other discomfort, airway damage, and other adverse effects. The hypoxic gases delivered could, in some configurations, be heated to at least about 30°C, but not more than about 50°C, 49°C, 48°C, 47°C, 46°C, 45°C, 44°C, or 43°C, such as about 31°C, 32°C, 33°C, 34°C, 35°C, 36°C, 37°C, 38°C, 39°C, 40°C, 41°C, 42°C, 43°C, or ranges incorporating any two of the aforementioned values. In some configurations, the gases can be heated to between about 31°C and about 50°C, between about 33°C and about 48°C, between about 35°C and about 46°C, between about 37°C and about 44°C, between about 39°C and about 42°C, between about 40°C and about 41°C, or about 41°C. In some configurations, the hypoxic gases are delivered at room temperature, such as between about 20°C and about 25°C, or other higher or lower temperatures, such as between about 15°C and about 30°C. The gases can also be humidified in some embodiments to prevent evaporation of moisture in the user's nasal passage and subsequently cool down too much to be effective. Humidified gases, for example, in the temperature ranges specified can in some embodiments carry enough latent heat to not only kill infections and reduce sickness times, but also act as a preventative measure, something which can be advantageous to athletes, among others. The hypoxic gases delivered could, in some configurations, have a dew point temperature of at least about 30°C, but not more than about 50°C, 49°C, 48°C, 47°C, 46°C, 45°C, 44°C, or 43°C, such as about 31°C, 32°C, 33°C, 34°C, 35°C, 36°C, 37°C, 38°C, 39°C, 40°C, 41°C, 42°C, 43°C, or ranges incorporating any two of the aforementioned values. In some configurations, the gases can have a dew point temperature of between about 31°C and about 50°C, between about 33°C and about 48°C, between about 35°C and about 46°C, between about 37°C and about 44°C, between about 39°C and about 42°C, between about 40°C and about 41°C, or about 41°C. In some configurations, the hypoxic gases can have a dew point temperature at room temperature, such as between about 20°C and about 25°C, or other higher or lower temperatures, such as between about 15°C and about 30°C. In some embodiments, the relative humidity of the hypoxic gas flow can be, for example, about or at least about 50*%*, 55*%*, 60*%*, 65*%*, 70*%*, 75*%*, 80*%*, 85*%*, 90*%*, 91*%*, 92*%*, 93*%*, 94*%*, 95*%*, 96*%*, 97*%*, 98*%*, 99*%*, or more, such as 100*%* or ranges incorporating any two of the aforementioned values. In some embodiments, the heating and/or humidification is not in an open room setup or configuration, and as such the heating and/or humidification is delivered through the discrete breathing conduit 16 directly connected to the user's nose and/or mouth.

The inspiratory conduit 16 and/or user interface 17 can be considered part of the flow therapy apparatus 10, or alternatively peripheral to it. The flow therapy apparatus 10, breathing conduit 16, and user interface 17 together can form a flow therapy system.

General operation of a flow therapy breathing apparatus 10 will now be described. The controller 13 can control the flow generator 11 to generate a gases flow of a desired flow rate, one or more valves to control mixing (e.g., hypoxic mixing) of air and nitrogen or other breathable gas, and/or the humidifier 12, if present, to humidify the gases flow to an appropriate temperature and/or humidity. In some embodiments, humidification can advantageously prevent the user's airways from drying out, which can be particularly advantageous during concomitant exercise due to fluid loss from sweating. The flow delivered could be at any desired flow rate. In some embodiments, the flow rate is about or at least about 10 liters/minute. In some embodiments, the flow rate is about, or less than about 10 liters/minute, such as 9, 8, 7, 6, 5, 4, 3, 2, or 1 liter/minute, or ranges incorporating any two of the aforementioned values.

As will be described in greater detail below, the apparatus 10 can use ultrasonic or other types of sensing to monitor characteristics of the gases in the flow. For example, the characteristics of the gases flow can include gases concentration, flow rate, or the like. The apparatus 10 can include additional sensors that can be in communication with the hardware processor. These sensors can include a flow rate sensor, a temperature sensor, a humidity sensor, a pressure sensor, or the like. Output of the additional sensors can be used for determining the characteristics of the gases flow, such as temperature, pressure, humidity, and the like. Output of the additional sensors can be used for correcting measurement of the characteristics of the gases flow by ultrasonic sensing. The gases flow can be directed out through the inspiratory conduit 16 and cannula 17 to the user. The cannula 17 may instead be any other user interface, such as a full face mask, nasal mask, nasal pillows mask, tracheostomy interface, or endotracheal tube. The controller 13 can also control a heating element in the humidifier 12 and/or the heating element 16a in the inspiratory conduit 16 to heat the gas to a desired temperature that achieves a desired level of therapy and/or level of comfort for the user. The controller 13 can be programmed with or can determine a suitable target temperature of the gases flow using one or more temperature sensors.

Additional sensors 3a, 3b, 3c, 20, 25, such as flow, temperature, humidity, and/or pressure sensors, can be placed in various locations in the flow therapy apparatus 10 and/or the inspiratory conduit 16 and/or cannula 17. The controller 13 can receive output from the sensors to assist it in operating the flow therapy apparatus 10 in a manner that provides suitable therapy. Providing suitable therapy can include meeting a user's peak inspiratory demand in some cases. The apparatus 10 can include a wireless data transmitter and/or receiver, or a transceiver 15 to enable the controller 13 to receive data signals 8 in a wireless manner from the operation sensors and/or to control the various components of the flow therapy apparatus 10. Additionally, or alternatively, the data transmitter and/or receiver 15 may deliver data to a remote server or enable remote control of the apparatus 10. The apparatus 10 can include a wired connection, for example, using cables or wires, to enable the controller 13 to receive data signals 8 from the operation sensors and/or to control the various components of the flow therapy apparatus 10. The transmitter can connect with a mobile device, such as a phone. The display, such as a graphic user interface (GUI) can be replicated on the mobile device's screen. Once connected to the apparatus 10, the mobile device can be used to control the device. The apparatus 10 can connect to the mobile device via a wireless connection, such as Bluetooth, WiFi, near field communication (NFC), 2G/3G/4G/5G, or other suitable wireless communications networks. The apparatus 10 can additionally or alternatively connect via a wired connection, such as USB, Ethernet, FireWire, serial port interface, or other suitable data transmission wires.

In some embodiments, one or more sensors (e.g., ultrasonic transducers as described herein) can be utilized to measure/verify the hypoxic gas composition (e.g., oxygen concentration) after, for example, nitrogen and ambient air have finished mixing. In some embodiments, at least one sensor on at least two of the ambient air inlet conduit, the nitrogen inlet conduit, and the final delivery conduit can be utilized to determine the flow rate of at least two gases. By inputting the flow rate of both inlet gases or one inlet gas and one total flow rate, along with the assumed oxygen concentrations of the inlet gases (e.g., 20.9*%* for ambient air, 0*%* for 100*%* pure enriched nitrogen), the oxygen concentration of the final gas composition can be calculated. As such, the hypoxic gas composition could include, for example, nitrogen enriched with between about 2*%* and about 18*%* oxygen (e.g., FdO₂), between about 2*%* and about 15*%* of oxygen, between about 10*%* and about 15*%* of oxygen, about 2*%*, 3*%*, 4*%*, 5*%*, 6*%*, 7*%*, 8*%*, 9*%*, 10*%*, 11*%*, 12*%*, 13*%*, 14*%*, 15*%*, 16*%*, 17*%*, 18*%*, 19*%*, 20*%* of oxygen, or ranges incorporating any of the aforementioned values. If inspiratory demand is met by the flow rate delivered to the user, FiO₂ can also be any of the aforementioned values. If inspiratory demand is not met, FiO₂ may be somewhat closer to the oxygen concentration in ambient air, as ambient air would be entrained around the cannula, which may reduce the nitrogen concentration in the gases inspired by the user.

In some embodiments, the hypoxic gas source itself could be made of a hypoxic gas of less than 100*%* purity. For example, the hypoxic gas source (e.g., enriched nitrogen and/or other hypoxic gases) could be at a purity of, for example, between about 82*%* and about 100*%*, such as between about 85*%* and about 90*%*, or about 82*%*, 83*%*, 84*%*, 85*%*, 86*%*, 87*%*, 88*%*, 89*%*, 90*%*, 91*%*, 92*%*, 93*%*, 94*%*, 95*%*, 96*%*, 97*%*, 89*%*, 99*%*, 100*%*, or ranges incorporating any of the aforementioned values. The oxygen concentration of the hypoxic gas source could be in some cases between about 2*%* and about 18*%* oxygen, between about 2*%* and about 15*%* of oxygen, between about 10*%* and about 15*%* of oxygen, about 2*%*, 3*%*, 4*%*, 5*%*, 6*%*, 7*%*, 8*%*, 9*%*, 10*%*, 11*%*, 12*%*, 13*%*, 14*%*, 15*%*, 16*%*, 17*%*, 18*%*, 19*%*, 20*%* of oxygen, or ranges incorporating any of the aforementioned values. Enriched nitrogen or another hypoxic gas at less than about 100*%* purity, such as 90*%* purity (with 10*%* oxygen), for example, can be advantageous from a safety perspective in preventing the FiO₂ from dipping below 10*%* (or another desired floor percentage), even in the event of a malfunction when mixing the nitrogen-enriched gas with ambient air.

In some embodiments, flow rate sensors can be placed at each location to allow for redundancy and testing that each sensor is working correctly by checking for consistency of readings. Any combination of the above methods, for example, could be implemented together to further allow for checking of consistency of results, along with failsafes if one or more sensors are not working correctly. Further sensors could be included (such as temperature and ambient pressure sensors) as disclosed herein to allow for correction of flow rate and/or gas concentration readings based on other factors.

Still referring to Figure 1, one, two, or more physiological sensors 99 can communicate with the data transmitter and/or receiver 15 to communicate data regarding physiological information of the user. The sensors 99 could be, for example, a pulse oximeter for measuring the oxygen saturation of a user's arterial blood, a heart rate sensor, blood pressure sensor, respiratory rate sensor, tidal volume sensor, ECG, heart rate variability sensor, and the like. Data received from the sensors 99 can also communicate with the controller 13 to be displayed on the user interface 14. Data from the sensors 99 can also be used by the controller 13 to provide closed loop feedback to modify various parameters of the device including oxygen concentration of the hypoxic gas concentration, flow rate, humidity, temperature, and the like as described in detail elsewhere herein.

### Overview of Example Flow Therapy Apparatus

The flow therapy apparatus may be any suitable type of apparatus, but in some configurations may deliver a high gases flow or high flow therapy (of e.g., air, nitrogen, a hypoxic gas mixture, or some combination thereof) to a user for altitude training, enhanced athletic performance, or other indications such as those discussed elsewhere herein. The gas can be or comprise nitrogen. The gas can comprise ambient air. The gas can comprise a blend of nitrogen and ambient air to create hypoxic gas compositions as noted elsewhere herein. The gas source could be a less than 100% pure enriched hypoxic gas such as a hypoxic mix of nitrogen and oxygen that is mixed with ambient air in the system that can be advantageous from a safety perspective in some cases as mentioned elsewhere herein. "High flow therapy" as used in this disclosure may refer to delivery of gases to a user at a flow rate of greater than or equal to about 10 liters/minute (10 LPM). In some configurations, "high flow" therapy refers to administration of gas to the airways of a patient at a relatively high flow rate. In some configurations, the relatively high flow rate meets or exceeds the peak inspiratory demand of the user. In other configurations, the high flow rate may not meet or exceed the peak inspiratory demand of the user. The flow rates used to achieve "high flow" may be any of the flow rates listed below. For example, in some configurations, for an adult patient "high flow therapy" may refer to the delivery of gases to a user at a flow rate of greater than or equal to about 10 litres per minute (10 LPM), such as between about 10 LPM and about 100 LPM, or between about 15 LPM and about 95 LPM, or between about 20 LPM and about 90 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. Gases delivered may comprise a percentage of nitrogen and oxygen. In some configurations, such as when the system is implementing a closed loop control algorithm as disclosed herein, delivery of hypoxic gases may not be limited to a high flow therapy and/or the inspiratory demand of the user may or may not be met by the flow rate.

In some embodiments, the device or system can allow for the user to change the flow rate during use, such as through interaction with a control, e.g., a user interface on the device. The flow rate may be varied, for example, by the controller varying the speed of the blower. This could be beneficial as the device could be used in different scenarios that require different flow rates. In some cases, flow rate could also oscillate in synchrony with breathing as long as a peak inspiratory demand of the user is being met. For example, if the user was to use the device while exercising then they could have an increased minute ventilation by way of an increased respiratory rate and/or increased tidal volume, so the flow rate could optionally be raised compared with when using the device at rest in order to meet increased peak inspiratory demand. In some embodiments, the device can allow for the user to change the oxygen concentration of the gases delivered via a control that modulates the flow of a hypoxic gas other than air, such as enriched nitrogen, from a hypoxic gas source into a conduit directly connected to the user, such as the nasal cannula.

The percentage of oxygen in the gases delivered may be, in embodiments where hypoxia is desired, such that the percentage of oxygen in the gas composition delivered to the user is less than that of typical room air (e.g., less than about 21*%*). In some embodiments, the percentage of oxygen in the gas composition is between about 10*%* and about 20.9*%*, or between about 11.9*%* and about 17.4*%*, or between about 13.8*%* and about 17.4*%*, or between about 15.7*%* and about 16.7*%*, or between about 15*%* and about 20*%*, or between about 13*%* and about 18*%*, or about or no more than about 10*%*, 10.5*%*, 11*%*, 11.5*%*, 12*%*, 12.5*%*, 13*%*, 13.5*%*, 14*%*, 14.5*%*, 15*%*, 15.5*%*, 16*%*, 16.5*%*, 17*%*, 17.5*%*, 18*%*, 18.5*%*, 19*%*, 19.5*%*, 20*%*, or 20.5*%*, or any ranges incorporating two of the aforementioned values. The percentage of oxygen in the inspired gas would be the same or greater than the aforementioned values, depending on whether inspiratory demand is met. In some embodiments, the percentage of nitrogen in the hypoxic gas composition is about or at least about 78.2*%*, 78.5*%*, 79*%*, 79.5*%*, 80*%*, 80.5*%*, 81*%*, 81.5*%*, 82*%*, 82.5*%*, 83*%*, 83.5*%*, 84*%*, 84.5*%*, 85*%*, 85.5*%*, 86*%*, 86.5*%*, 87*%*, 87.5*%*, 88*%*, 88.5*%*, 89*%*, 89.5*%*, 90*%*, or more, or any ranges incorporating two of the aforementioned values. The percentage of nitrogen in the inspired gas would be the same or less than the aforementioned values, depending on whether inspiratory demand is met. In some embodiments, the percentage of inspired or delivered nitrogen, oxygen, and/or other gases could be any of the preceding values or ranges, or others as disclosed elsewhere herein.

High flow hypoxic gas therapy can be effective in meeting or exceeding the user's peak inspiratory demand, delivering nitrogen enriched air (and therefore O₂ diminished air) and using a nasal high flow system configured for a variety of medical and non-medical applications, including but not limited to simulated altitude training, enhanced athletic performance, or other indications. Not to be limited by theory, in some embodiments systems and methods as disclosed herein could be used to treat or prevent hypertension, obesity, hyperlipidemia, prediabetes or impaired glucose tolerance, diabetes mellitus type I or type II, metabolic syndrome, mitochondrial regeneration, aging, fatigue, inflammatory diseases, anemia, or other conditions. Additionally, high flow therapy may generate a flushing effect in the nasopharynx such that the anatomical dead space of the upper airways is flushed completely or substantially completely by the high incoming gases flow. This can create a reservoir of fresh gas available of each and every breath of the exact desired composition (e.g., hypoxic composition), and prevent entrainment of ambient air that would alter the composition of the gas as well as minimizing re-breathing of carbon dioxide. These aforementioned features can work together to give increased consistency and control of the hypoxic solution that the user breathes, regardless of factors such as breath rate and volume.

The user interface may be in some embodiments a non-sealing interface, which advantageously prevents barotrauma (e.g. tissue damage to the lungs or other organs of the respiratory system due to difference in pressure relative to the atmosphere). The user interface may be a nasal cannula with a manifold and nasal prongs, and/or a face mask, and/or a nasal pillows mask, and/or a nasal mask, and/or a tracheostomy interface, or any other suitable type of user interface.

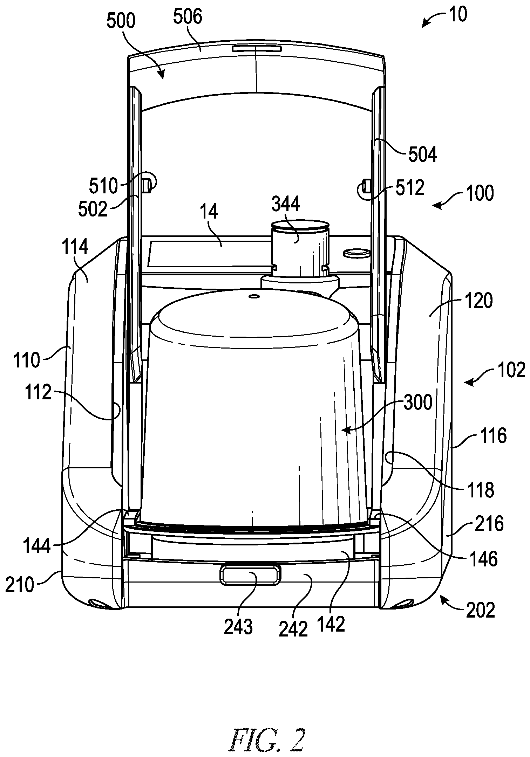

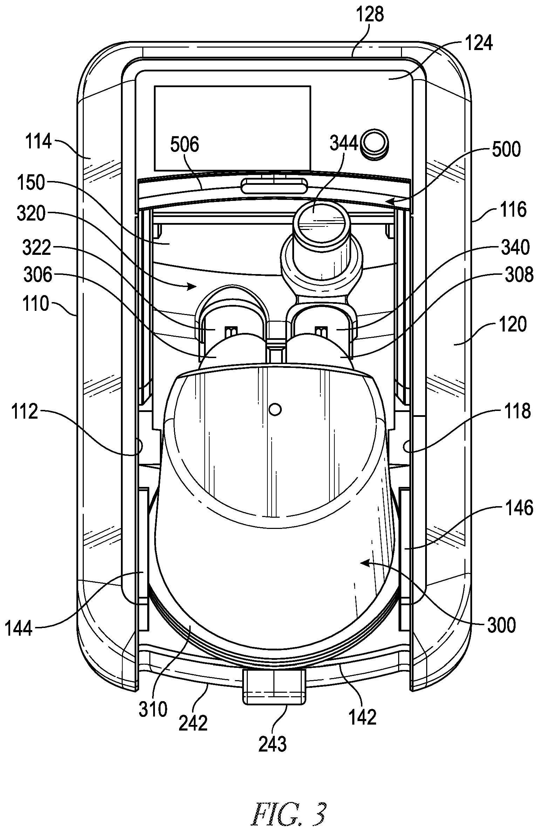

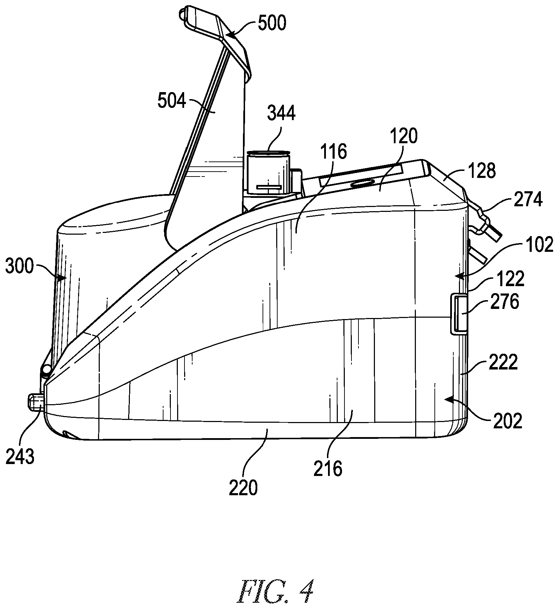

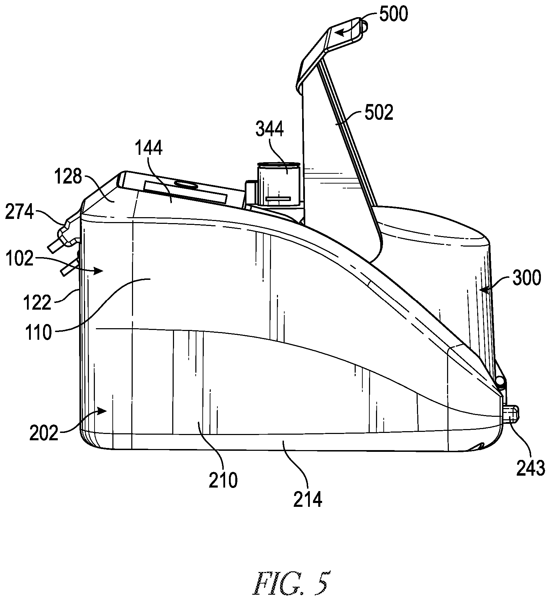

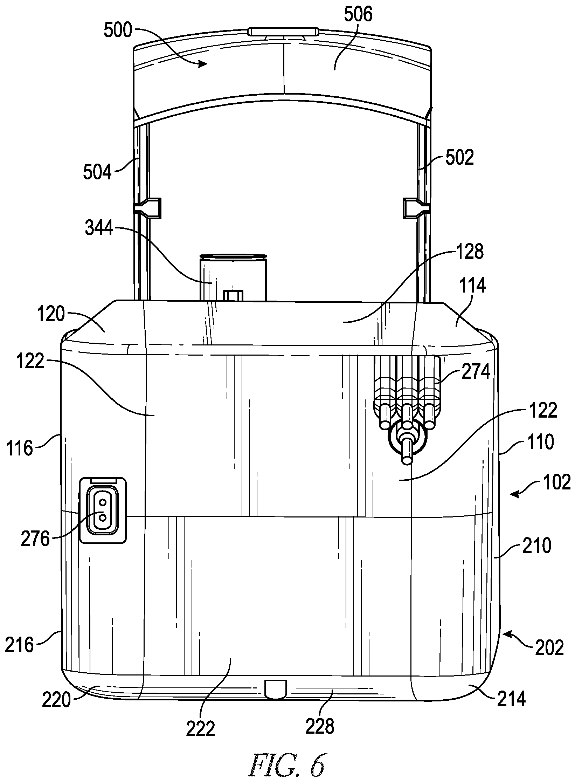

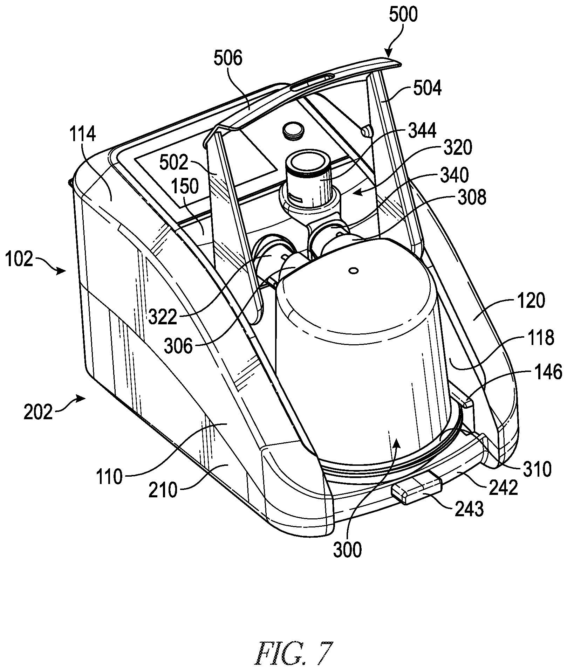

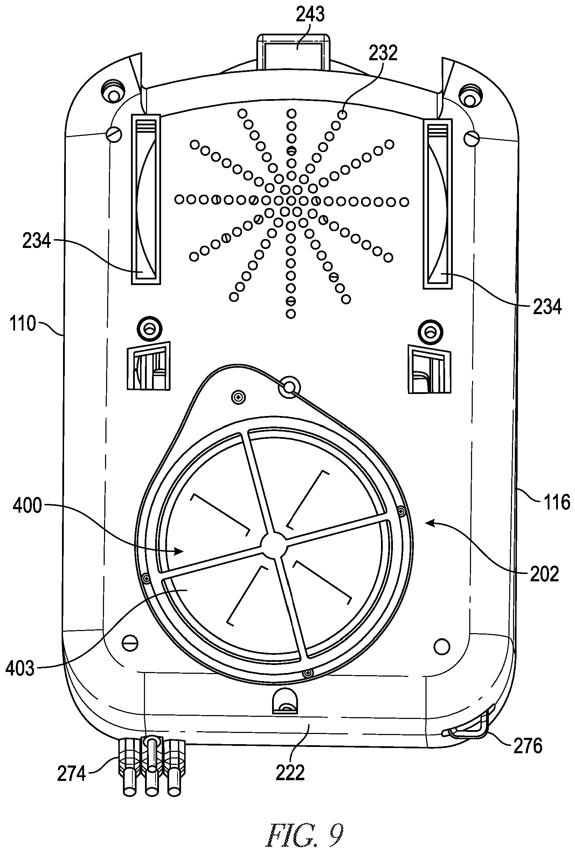

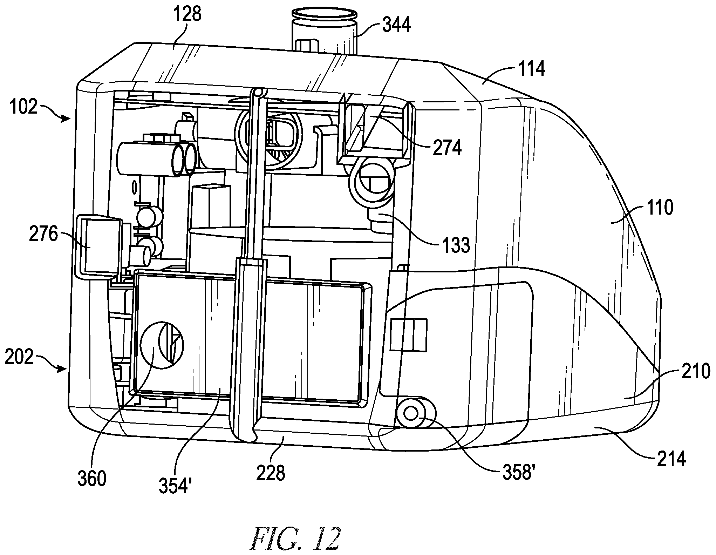

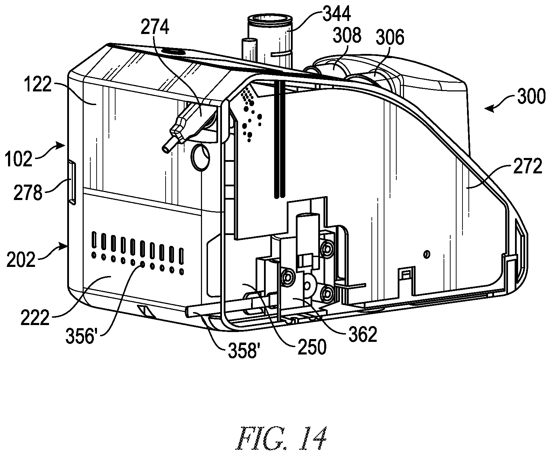

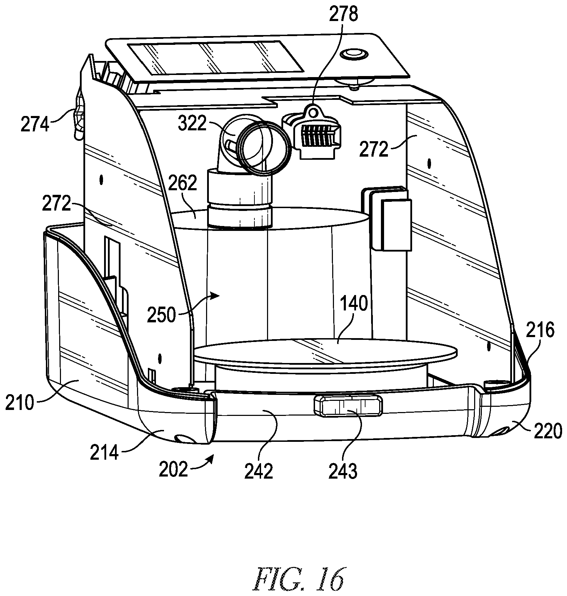

Some examples of the flow therapy apparatus are described herein. Figures 2 to 17B show an example flow therapy apparatus 10 having a main housing 100. The main housing 100 has a main housing upper chassis 102 and a main housing lower chassis 202.

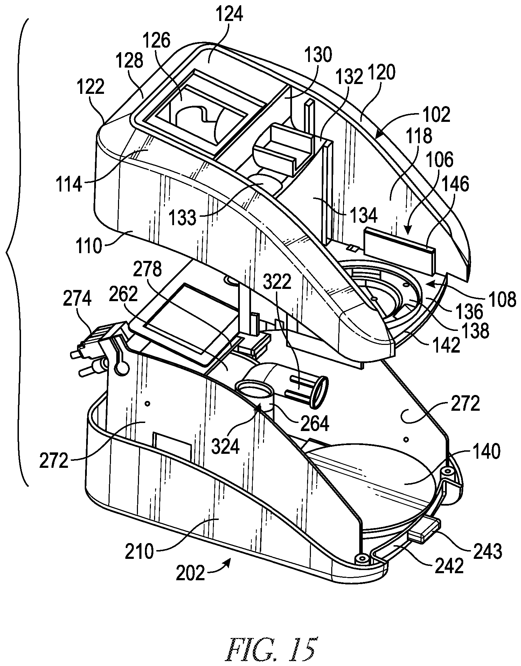

The main housing upper chassis 102 has a peripheral wall arrangement 106 (see Figure 15). The peripheral wall arrangement defines a humidifier or humidification chamber bay 108 for receipt of a removable humidification chamber 300. The removable humidification chamber 300 contains a suitable liquid such as water for humidifying gases that can be delivered to a user.

In the form shown, the peripheral wall arrangement 106 of the main housing upper chassis 102 can include a substantially vertical left side outer wall 110 that is oriented in a front-to-rear direction of the main housing 100, a substantially vertical left side inner wall 112 that is oriented in a front-to-rear direction of the main housing 100, and an interconnecting wall 114 that extends between and interconnects the upper ends of the left side inner and outer walls 110, 112. The main housing upper chassis 102 can further include a substantially vertical right side outer wall 116 that is oriented in a front-to-rear direction of the main housing 100, a substantially vertical right side inner wall 118 that is oriented in a front-to-rear direction of the main housing 100, and an interconnecting wall 120 that extends between and interconnects the upper ends of the right side inner and outer walls 116, 118. The interconnecting walls 114, 120 are angled towards respective outer edges of the main housing 100, but can alternatively be substantially horizontal or inwardly angled.

The main housing upper chassis 102 can further include a substantially vertical rear outer wall 122. An upper part of the main housing upper chassis 102 can include a forwardly angled surface 124. The surface 124 can have a recess 126 for receipt of a display and user interface module 14. The display can be configured to display characteristics of sensed gas(es) in real time, such as the oxygen concentration, temperature, humidity, and/or other characteristics. In some embodiments, the oxygen concentration could be displayed as an altitude above sea level (e.g., in meters or feet) that would result in an equivalent partial pressure of oxygen. Conversions between FiO₂ and equivalent altitude could be done by the device by assuming an ambient oxygen concentration of, for example, about 20.9%. An interconnecting wall 128 can extend between and interconnect the upper end of the rear outer wall 122 and the rear edge of the surface 124.

A substantially vertical wall portion 130 can extend downwardly from a front end of the surface 124. A substantially horizontal wall portion 132 can extend forwardly from a lower end of the wall portion 130 to form a ledge. A substantially vertical wall portion 134 can extend downwardly from a front end of the wall portion 132 and terminate at a substantially horizontal floor portion 136 of the humidification chamber bay 108. The left side inner wall 112, right side inner wall 118, wall portion 134, and floor portion 136 together can define the humidification chamber bay 108. The floor portion 136 of the humidification chamber bay 108 can have a recess 138 to receive a heater arrangement such as a heater plate 140 or other suitable heating element(s) for heating liquid in the humidification chamber 300 for use during a humidification process.

The main housing lower chassis 202 can be attachable to the upper chassis 102, either by suitable fasteners or integrated attachment features such as clips for example. The main housing lower chassis 202 can include a substantially vertical left side outer wall 210 that is oriented in a front-to-rear direction of the main housing 100 and is contiguous with the left side outer wall 110 of the upper chassis 102, and a substantially vertical right side outer wall 216 that is oriented in a front-to-rear direction of the main housing 100 and is contiguous with the right side outer wall 116 of the upper chassis 102. The main housing lower chassis 202 can further include a substantially vertical rear outer wall 222 that is contiguous with the rear outer wall 122 of the upper chassis 102.

The lower housing chassis 202 can have a lip 242 that is contiguous with the lip 142 of the upper housing chassis 102, and also forms part of the recess for receiving the handle portion 506 of the lever 500. The lower lip 242 can include a forwardly directed protrusion 243 that acts as a retainer for the handle portion 506 of the lever 500.

An underside of the lower housing chassis 202 can include a bottom wall 230. Respective interconnecting walls 214, 220, 228 can extend between and interconnect the substantially vertical walls 210, 216, 222 and the bottom wall 230. The bottom wall 230 can include a grill 232 comprising a plurality of apertures to enable drainage of liquid in case of leakage from the humidification chamber 300 (e.g. from spills). The bottom wall 230 additionally can include elongated forward-rearward oriented slots 234. The slots 234 can additionally enable drainage of liquid in case of leakage from the humidification chamber 300, without the liquid entering the electronics housing. In the illustrated configuration, the slots 234 can be wide and elongate relative to the apertures of the grill 232 to maximize the drainage of liquid.

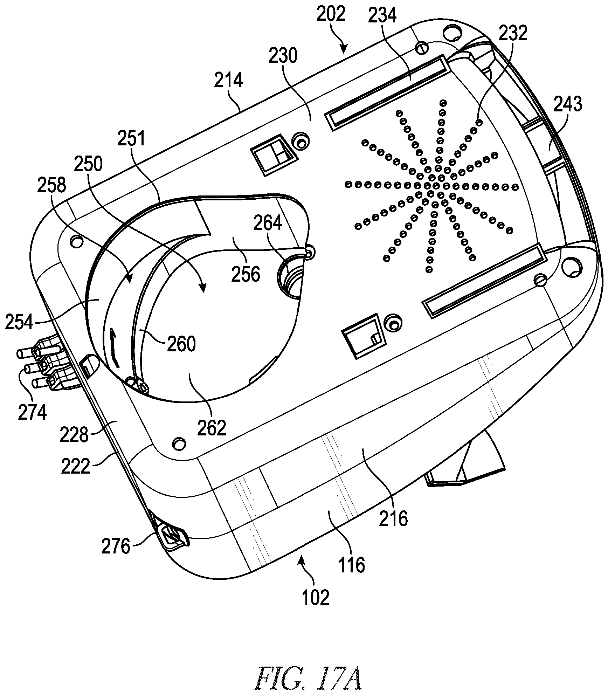

As shown in Figure 17A to 17B, the lower chassis 202 can have a motor recess 250 for receipt of a motor/sensor module. The motor/sensor module may be non-removable from the main housing 100. The motor/sensor module may be removable from the main housing 100, as illustrated in Figures 17A-17B. A recess opening 251 can be provided in the bottom wall 230 adjacent a rear edge thereof, for receipt of a removable motor/sensor module. A continuous, gas impermeable, unbroken peripheral wall 252 can be integrally formed with the bottom wall 230 of the lower chassis 202 and extend upwardly from the periphery of the opening 251. A rearward portion 254 of the peripheral wall 252 has a first height, and a forward portion 256 of the peripheral wall 252 has a second height that is greater than the first height. The rearward portion 254 of the peripheral wall 252 terminates at a substantially horizontal step 258, which in turn terminates at an upper auxiliary rearward portion 260 of the peripheral wall 252. The forward portion 256 and upper auxiliary rearward portion 260 of the peripheral wall 252 terminate at a ceiling 262. All of the walls and the ceiling 262 can be continuous, gas impermeable, and unbroken other than the gases flow passage. Therefore, the entire motor recess 250 can be gas impermeable and unbroken, other than the gases flow passage.

The motor/sensor module may be insertable into the recess 250 and attachable to the lower chassis 202. Upon insertion of the motor/sensor module into the lower chassis 202, the gases flow passage tube 264 can extend through the downward extension tube 133 and be sealed by the soft seal.

The humidification chamber 300 can be fluidly coupled to the apparatus 10 in a linear slide-on motion in a rearward direction of the humidification chamber 300 into the chamber bay 108, from a position at the front of the housing 100 in a direction toward the rear of the housing 100. A gases outlet port 322 can be in fluid communication with the motor.

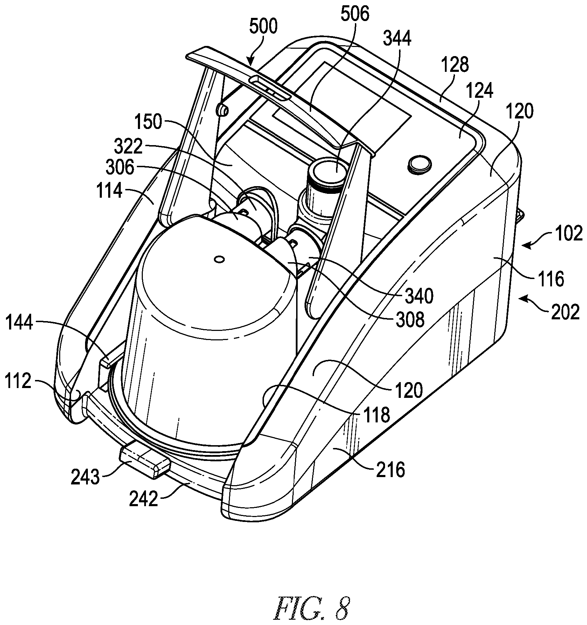

A gases inlet port 340 (humidified gases return) as shown in Figure 8 can include a removable L-shaped elbow. The removable elbow can further include a user outlet port 344 for coupling to the inspiratory conduit 16 to deliver gases to the user interface 17. The gases outlet port 322, gases inlet port 340, and user outlet port 344 each can have soft seals such as O-ring seals or T-seals to provide a sealed gases passageway between the apparatus 10, the humidification chamber 300, and the inspiratory conduit 16.

The humidification chamber gases inlet port 306 can be complementary with the gases outlet port 322, and the humidification chamber gases outlet port 308 can be complementary with the gases inlet port 340. The axes of those ports can be parallel to each other to enable the humidification chamber 300 to be inserted into the chamber bay 108 in a linear movement.

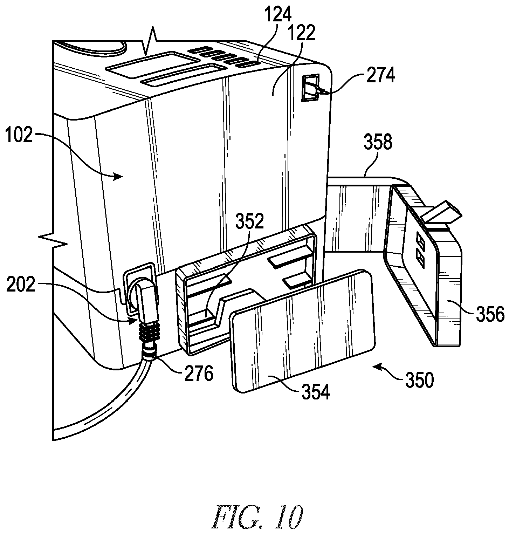

The apparatus 10 can have air and nitrogen (or alternative auxiliary gas) inlets in fluid communication with the motor to enable the motor to deliver air, nitrogen-enhanced air to form a hypoxic mixture, or a suitable mixture thereof to the humidification chamber 300 and thereby to the user. As shown in Figure 10, the apparatus 10 may have a combined air/nitrogen (or alternative auxiliary gas) inlet arrangement 350. This arrangement can include a combined air/nitrogen port 352 into the housing 100, a filter 354, and a cover 356 with a hinge 358. In other embodiments, a gases tube can extend laterally or in another appropriate direction and be in fluid communication with a nitrogen source. The port 352 can be fluidly coupled with the motor 402. For example, the port 352 may be coupled with the motor/sensor module 400 via a gases flow passage between the port 352 and an inlet aperture or port in the motor/sensor module 400, which in turn would lead to the motor.

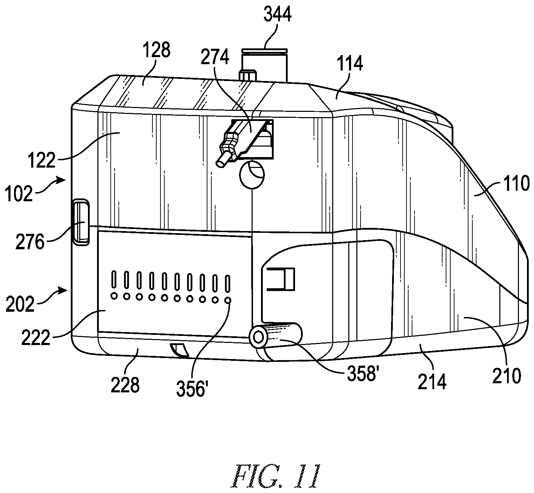

The apparatus 10 may have the arrangement shown in Figures 11 to 14 to enable the motor to deliver air, nitrogen (or alternative auxiliary gas), or a suitable mixture thereof to the humidification chamber 300 and thereby to the user. This arrangement can include an air inlet 356' in the rear wall 222 of the lower chassis 202 of the housing 100. The air inlet 356' comprises a rigid plate with a suitable grill arrangement of apertures and/or slots. Sound dampening foam may be provided adjacent the plate on the interior side of the plate. An air filter box 354' can be positioned adjacent the air inlet 356' internally in the main housing 100, and include an air outlet port 360 to deliver filtered air to the motor via an air inlet port 404 in the motor/sensor module 400. The air filter box 354' may include a filter configured to remove particulates (e.g. dust) and/or pathogens (e.g. viruses, fungi, or bacteria) from the gases flow. A soft seal such as an O-ring seal can be provided between the air outlet port 360 and air inlet port 404 to seal between the components. The apparatus 10 can include a separate nitrogen inlet port 358' positioned adjacent one side of the housing 100 at a rear end thereof, the nitrogen port 358' for receipt of nitrogen from a nitrogen source such as a tank or source of piped nitrogen. The nitrogen inlet port 358' can optionally be in fluid communication with a valve 362. The valve 362 can suitably be a solenoid valve that enables the control of the amount of nitrogen that is added to the gases flow that is delivered to the humidification chamber 300. The nitrogen port 358' and valve 362 may be used with other (e.g., other than nitrogen) auxiliary gases to control the addition of other auxiliary gases to the gases flow. The other auxiliary gases may include any one or more of a number of gases useful for gas therapy, including but not limited to heliox, oxygen, nitrogen, nitric oxide, carbon dioxide, argon, helium, methane, sulfur hexafluoride, and combinations thereof. In some embodiments a valve need not be present, and nitrogen flow can be manually controlled at the nitrogen source. The oxygen concentration of the hypoxic gases flow can be read from the user interface, and the amount of nitrogen titrated at the nitrogen source accordingly depending on the desired result.

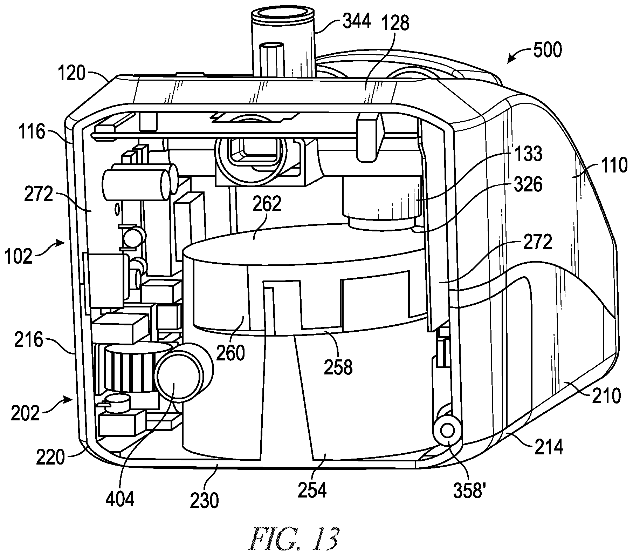

As shown in Figures 13 to 16, the lower housing chassis 202 can include suitable electronics boards 272, such as sensing circuit boards. The electronics boards can be positioned adjacent respective outer side walls 210, 216 of the lower housing chassis 202. The electronics boards 272 can contain, or can be in electrical communication with, suitable electrical or electronics components, such as but not limited to microprocessors, capacitors, resistors, diodes, operational amplifiers, comparators, and switches. Sensors may be used with the electronic boards 272. Components of the electronics boards 272 (such as but not limited to one or more microprocessors) can act as the controller 13 of the apparatus.

One or both of the electronics boards 272 can be in electrical communication with the electrical components of the apparatus 10, including the display unit and user interface 14, motor, valve 362, and the heater plate 140 to operate the motor to provide the desired flow rate of gases, operate the humidifier 12 to humidify and heat the gases flow to an appropriate level, and supply appropriate quantities of nitrogen (or quantities of an alternative auxiliary gas) to the gases flow.

The electronics boards 272 can be in electrical communication with a connector arrangement 274 projecting from the rear wall 122 of the upper housing chassis 102. The connector arrangement 274 may be coupled to an audible, visual, tactile, or other alarm, pulse oximetry port, and/or other suitable accessories. The electronics boards 272 can also be in electrical communication with an electrical connector 276 that can also be provided in the rear wall 122 of the upper housing chassis 102 to provide mains or battery power to the components of the apparatus 10.

As mentioned above, operation sensors, such as flow, temperature, humidity, and/or pressure sensors can be placed in various locations in the flow therapy apparatus 10 and/or the inspiratory conduit 16 and/or cannula 17. The electronics boards 272 can be in electrical communication with those sensors. Output from the sensors can be received by the controller 13, to assist the controller 13 to operate the flow therapy apparatus 10 in a manner that provides optimal therapy, including optionally meeting peak inspiratory demand.

As outlined above, the electronics boards 272 and other electrical and electronic components can be pneumatically isolated from the gases flow path to improve safety. The sealing also prevents water ingress.

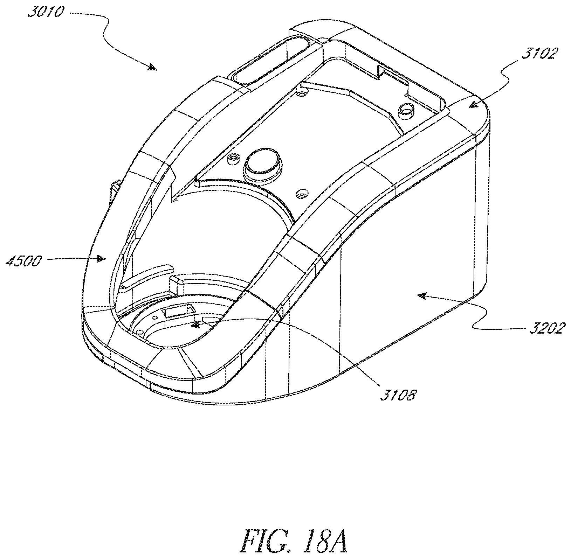

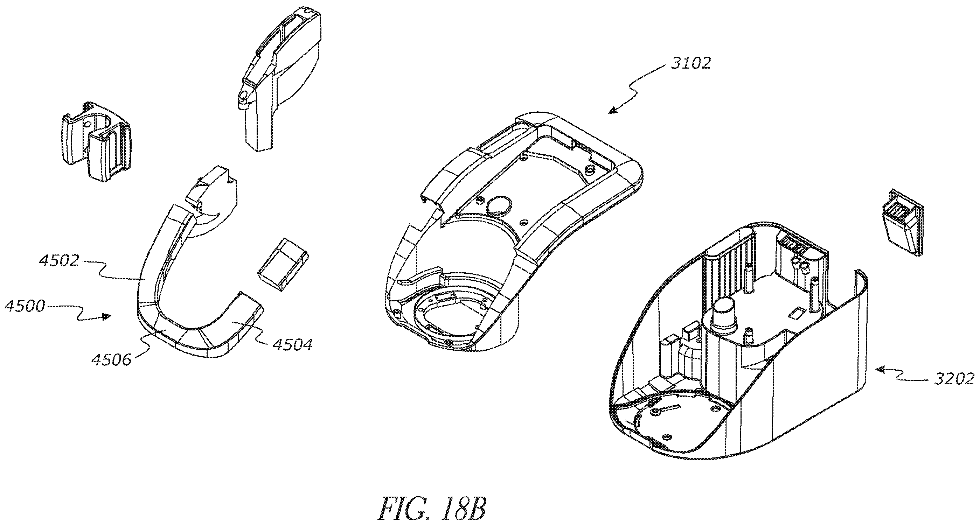

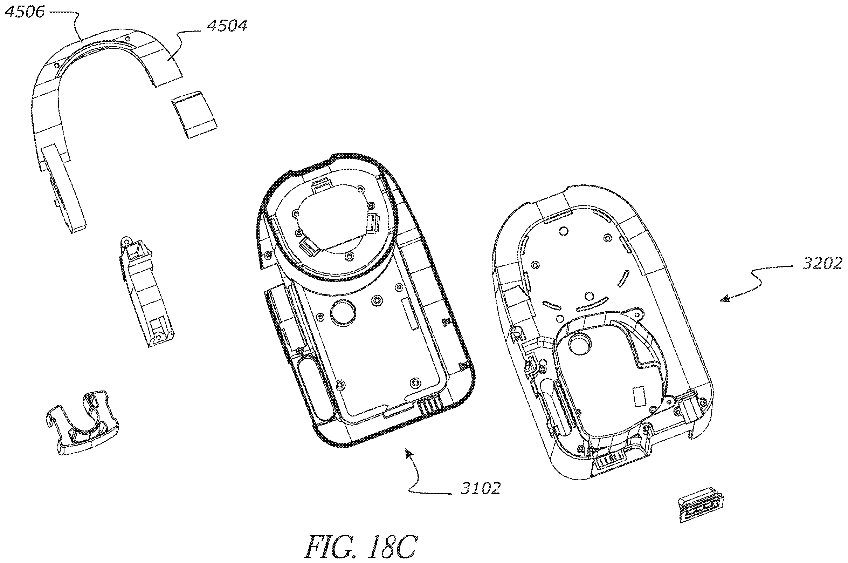

Figures 18A-E illustrate another flow therapy apparatus 3010 including a main housing having a main housing upper chassis 3102 and a main housing lower chassis 3202. The flow therapy apparatus 3010 can further include a humidification chamber bay 3108 for receipt of a removable humidification chamber. The flow therapy apparatus 3010 may have any of the features and/or functionality described herein in relation to the flow therapy apparatus 10, but those features are not repeated here for simplicity. Similarly, the features and/or functionality of the flow therapy apparatus 3010 may be used in the other apparatus described herein.

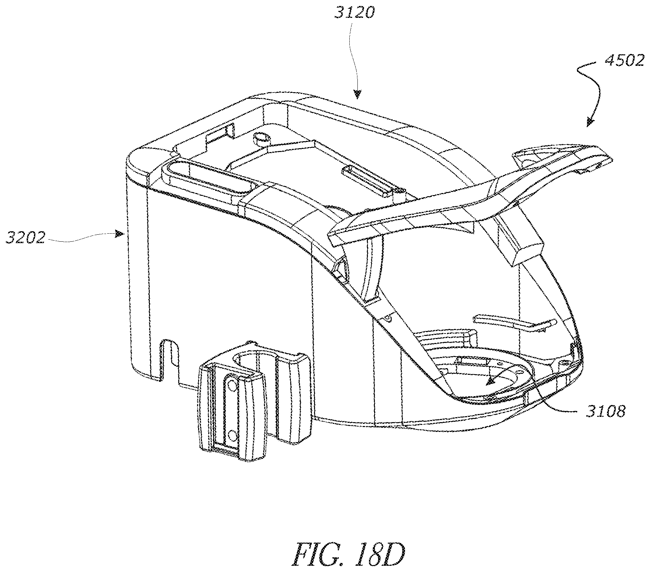

The flow therapy apparatus 3010 can have a single-sided handle/lever 4500. That is, only one side of the handle/lever 4500 is movably connected relative to the main housing of the flow therapy apparatus 3010, whereas there is no pivot connection of the other side of the handle/lever 4500 to the main housing. As shown in Figure 18D, a left side of the handle/lever 4500 is pivotally connected relative to the main housing. However, it is possible to have only the right side pivotally connected to the main housing. The handle/lever 4500 is pivotally and translationally connected to the main housing, so that the handle/lever 4500 moves on a path having a varying radius relative to the main housing.

A terminal part of the handle/lever 4500 can have a cross-member handle portion 4506 that interconnects forward ends of a left side arm 4502 and a right side member 4504 and forms an engagement region for grasping by a user's fingers. When the handle 4500 is in the raised position as shown in Figure 18D for example, the cross-member 4506 can act as a carrying handle for the flow therapy apparatus 3010. When the handle is in the fully raised position, the cross member 4506 can be positioned generally above and generally in line with the centre of gravity of the flow therapy apparatus 3010 (including the liquid chamber). The liquid chamber can be inserted into or removed from the humidification chamber bay 3108 when the handle/lever 4500 is raised. When the handle/lever 4500 is in the lowered position, it can inhibit or prevent removal of the liquid chamber from the humidification chamber bay 108.

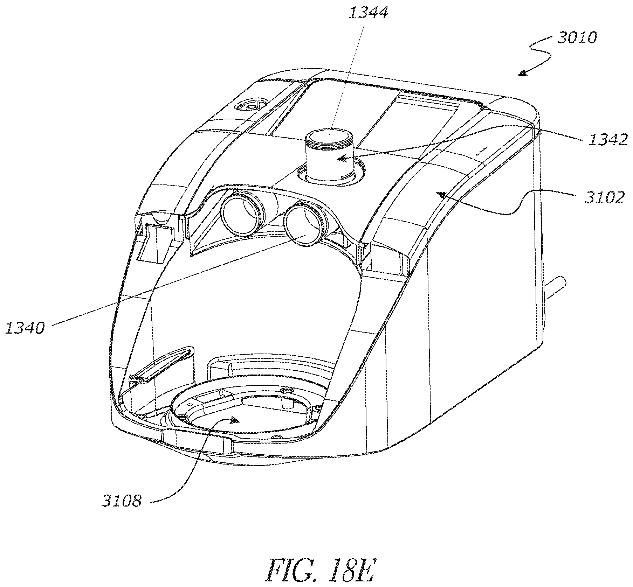

Figure 18E illustrates the flow therapy apparatus 3010 without the handle/lever. As shown in Figure 18E, a removable gases flow tube in the form of a removable elbow 1342 can be used in the flow therapy apparatus 3010. The elbow 1342 can receive humidified gases from the liquid chamber at an inlet port 1340 and direct the humidified gases to an outlet port 1344 toward the user interface through the user breathing conduit.

Similar to the flow therapy apparatus 10, the lower chassis 3202 of the flow therapy apparatus 3010 can have a motor recess for receiving a motor/sensor module. The motor/sensor module can include a blower, which entrains room air to deliver to a user. The gases can be mixed prior to entering the sensor module. The blower can be a mixer for mixing the gases before the gases enter a sensing chamber of the sensor module. The apparatus can include an integrated or separate gas mixer in some cases. The separate gas mixer can be positioned before or after the blower. Nitrogen can be entrained after the blower and the separate gas mixer can be used to mix the nitrogen and air following entrainment. The controller can increase or decrease a flow rate of the gases flowing through the flow therapy apparatus by controlling a motor speed of the blower.

### Control System

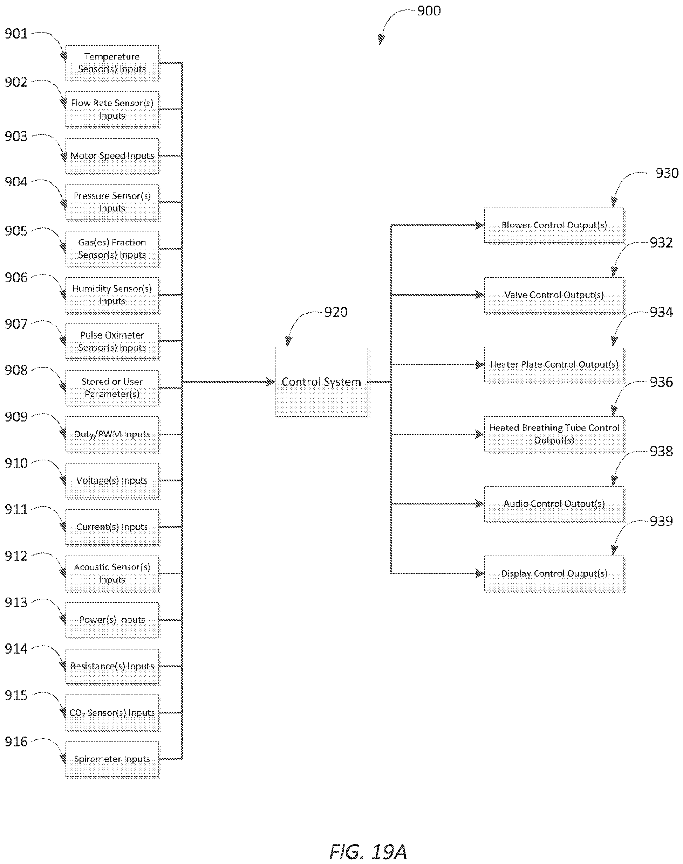

FIG. 19A illustrates a block diagram 900 of an example control system 920 that can detect user conditions and control operation of the flow therapy apparatus including the gas source. The control system 920 can manage a flow rate of the gas flowing through the flow therapy apparatus as it is delivered to a user. For example, the control system 920 can increase or decrease the flow rate by controlling an output of a motor speed of the blower (hereinafter also referred to as a "blower motor") 930 or an output of a valve 932 in a blender. The control system 920 can automatically determine a set value or a personalized value of the flow rate for a particular user as discussed below. The flow rate can be optimized by the control system 920 to improve user comfort and therapy.

The control system 920 can also generate audio and/or display/visual outputs 938, 939. For example, the flow therapy apparatus can include a display and/or a speaker. The display can indicate to the user, physician, trainer, or other party any warnings or alarms generated by the control system 920. The display can also indicate control parameters that can be adjusted by the user or other individual. For example, the control system 920 can automatically recommend a flow rate for a particular user. The control system 920 can also determine a respiratory state of the user, including but not limited to generating a respiratory rate of the user, and send it to the display.

In some embodiments, a physiological parameter, such as SpO₂ of a user for example, could additionally or alternatively be fed into an alarm system. The alarm system could monitor the user's SpO₂, and have set responses if it were to fall below a certain level. The device could additionally or alternatively have alarms for FiO₂ values, assuming that the user's inspiratory demand is met, and that FdO₂ is equal to FiO₂. As described above, if the user's inspiratory demand is not met, the actual FiO₂ value would be higher than the assumed value. The alarms would be on the basis of the measured oxygen concentration in the unit, which is FdO₂. The device would alarm on the basis of FdO₂ and assume that the parameter being measured is FiO₂. This could affect therapy, but not affect safety. Responses from the system could include visual and/or audible alarms, reducing the nitrogen being supplied, setting the nitrogen flow to a predetermined value, setting the nitrogen flow to achieve a specific FiO₂ or SpO₂, completely shutting off the nitrogen supply, shutting off all flow completely or any combination of these responses.

The system could have multiple thresholds, where different responses or sets of responses are triggered at different SpO₂ and/or FiO₂ readings. As subsequent thresholds are crossed, different responses could occur in place of the previous responses, in addition to the previous responses, and/or act as a variation of a previous response (e.g. an audible alarm changes in volume or tone).

In some embodiments, the device could alarm for at least the FiO₂ when controlling SpO₂, and vice versa, as this could mean the user is prevented from reaching dangerous levels of both SpO₂ and FiO₂, as one is bound by the control limits of the device and the other is bound by alarm limits. It would also have added safety through redundancy in the event of a faulty reading of one of the SpO₂ or FiO₂ sensors.

Alarm thresholds could include time factors. These would allow for the device to not be triggered by brief false readings, such as requiring a threshold to be about or at least about 5, 10, 15, 20, 25, 30, or more seconds for example. They could also allow for escalation in response of the device based on length of time at a certain value (for example, going from a visual to audible alarm, or a louder or more prominent alarm). They could also allow for alarms to be set off after a set amount of time (for example, alarming at SpO₂ readings that are safe for a short exposure but become dangerous after a longer duration of exposure, such as, for example, at about or at least about 30, 45, 60, 75, 90, 120, 150, 180, 240, 300 seconds, or more).

Some non-limiting examples of values at which an SpO₂ reading could trigger an alarm could include below about 94% (as this is the lower limit of what is considered a healthy SpO₂ reading at sea level), and/or below about 85% (as this is typically the value below which negative side effects begin to occur), and/or below about 80% (as this is the value that is typically regarded as the point where altitude training becomes dangerous), and/or below about 75% (as this is the point where therapy should be stopped immediately), and/or below about 65% (as this is the point where the user would likely have impaired mental function and judgement), and/or below about 55% (as this is the point where the user would likely lose consciousness).

Some values at which FiO₂/equivalent altitude readings could trigger a response from the system could include about 15.7% (that is, about 2500m) as this is typically the upper altitude limit of an extended exposure therapy program, and/or about 13.8% (that is, about 3500m) as this is typically the upper altitude limit of any therapy program, and/or about 11.9% (that is, about 4800m) as this is the limit of what is typically considered safe without medical supervision, and/or about 10.2% (that is, about 5950m) as this represents the highest recorded permanently tolerable altitude, and/or about 7.7% (that is, about 8000m) as this is commonly referred to as the "death zone" where the oxygen concentration is insufficient to sustain human life.

The device could additionally or alternatively have one or more alarm limits for SpO₂ and/or FiO₂ that change in response to one or more inputs. The one or more inputs could include an FiO₂ reading, an SpO₂ reading, a heart rate measurement, a respiratory rate measurement, an input by the user of their desired therapy program, a sensed or user input indication that the user is exercising, an amount of time the user has been using the device, and/or one or more user characteristics (this could include age, height, weight, gender, activity/fitness level, experience with altitude training). The indication of user exercise in particular could come from one or a combination of the other inputs listed, and could be transmitted as qualitative signal (e.g., either exercising or not exercising) or as a quantitative signal (e.g., exercise intensity level).

The relationship between one or more alarm limits and one or more inputs could be one that changes dynamically (e.g., as age increases the alarm trigger threshold for FiO₂ steadily increases). Additionally, or alternatively, one or more alarm limits could change by a set amount in response to one or more inputs. The change in one or more limits could occur at one or more thresholds of a quantitative signal (e.g., the alarm threshold for SpO₂ increases by a certain amount after a specific amount of time that user has been using the device) and/or in response to a qualitative signal (e.g., the FiO₂ alarm threshold jumps to a higher value when the user indicates that they are beginning exercise).

Having alarm limits that vary on a range of conditions can allow the user to safely employ a training regime that allows them to reach their maximum potential. While alarm limits can be applied in a blanket "one fit all" approach in some embodiments, this may not be desirable in some implementations because in order for the system to be safe for all or most users in all or most scenarios the device may become overly safe (or not challenging enough) for others, to the point where certain users are not able to gain maximum benefit from the device. The system would also be able to provide alarms for readings, particularly SpO₂ readings, that while potentially safe are abnormal given the current parameters provided. For example, if a user had an SpO₂ reading of less than 94% while at rest and breathing normoxic gas, then the device could alarm to tell the user that his or her resting SpO₂ is abnormally low, and the user should consult a medical professional before commencing with an altitude training program.

In some embodiments, any alarms that are capable of changing based off various inputs would be coupled with one or more fixed alarms (such as the ones listed above) that provide absolute limits regardless of any input.

A similar methodology to the above could be additionally or alternatively applied in a similar way to the ranges for FiO₂ and/or SpO₂ that the user can attempt to control to. As above, this could allow the user to use the device in a way that is individually tailored to be both optimally effective and safe based on the parameters provided. It could allow for access to a greater range of training levels while avoiding potentially dangerous misuse by the user. Optionally, a fixed upper limit for the device could be coupled with a variable lower limit, or vice versa. The device could have one or more variable limits paired with a fixed limit that the variable limit could not exceed.

Table 1 below lists various non-limiting flow rates of nitrogen to illustrate what would roughly be required to achieve various oxygen concentrations. Preferably a value for total flow would be selected, and this would remain constant regardless of changes to nitrogen flow rate and/or FiO₂, unless a new total flow rate is selected. If the total flow rate in the device is adjusted, a proportional change in the nitrogen flow rate would be required to maintain the same FiO₂.

**TABLE 1**

| Total flow L/min | Nitrogen flowrate (L/min) | FiO₂ (assuming inspiratory demand is met) |
|---|---|---|
| 30 | 0 | 21.0*%* |
| 30 | 1 | 20.2*%* |
| 30 | 2 | 19.4*%* |
| 30 | 3 | 18.7*%* |
| 30 | 4 | 17.9*%* |
| 30 | 5 | 17.1*%* |
| 30 | 6 | 16.3*%* |
| 30 | 7 | 15.6*%* |
| 30 | 8 | 14.8*%* |
| 30 | 9 | 14.0*%* |
| 30 | 10 | 13.2*%* |

The control system 920 can change heater control outputs to control one or more of the heating elements (for example, to maintain a temperature set point of the gas delivered to the user). The control system 920 can also change the operation or duty cycle of the heating elements. The heater control outputs can include heater plate control output(s) 934 and heated breathing tube control output(s) 936.

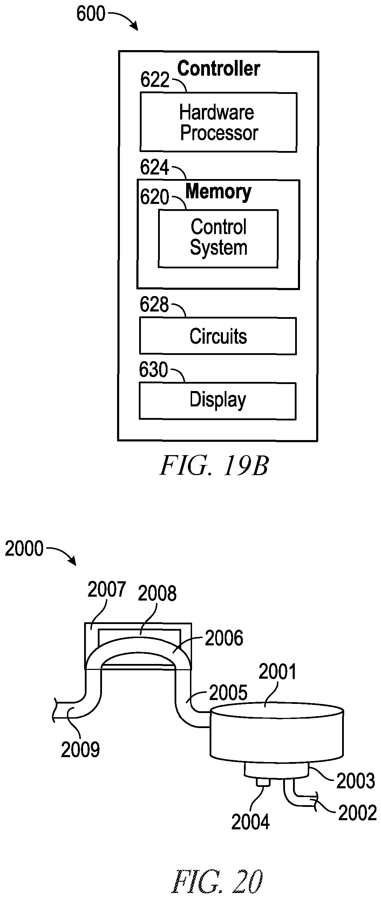

The control system 920 can determine the outputs 930-939 based on one or more received inputs 901-916. The inputs 901-916 can correspond to sensor measurements received automatically by the controller 600 (shown in FIG. 19B). The control system 920 can receive sensor inputs including but not limited to temperature sensor(s) inputs 901, flow rate sensor(s) inputs 902, motor speed inputs 903, pressure sensor(s) inputs 904, gas(s) fraction sensor(s) inputs 905, humidity sensor(s) inputs 906, pulse oximeter (for example, SpO₂) sensor(s) inputs 907, stored or user parameter(s) 908, duty cycle or pulse width modulation (PWM) inputs 909, voltage(s) inputs 910, current(s) inputs 911, acoustic sensor(s) inputs 912, power(s) inputs 913, resistance(s) inputs 914, CO₂ sensor(s) inputs 915, and/or spirometer inputs 916. The control system 920 can receive inputs from the user or stored parameter values in a memory 624 (shown in FIG. 19B). The control system 920 can dynamically adjust flow rate for a user over the time of their therapy. The control system 920 can continuously detect system parameters and user parameters. A person of ordinary skill in the art will appreciate based on the disclosure herein that any other suitable inputs and/or outputs can be used with the control system 920.

In some embodiments, a target oxygen concentration could be selected, and the difference between this and the measured oxygen concentration could be fed into a controller for the valve, which would open and close to alter the supply of nitrogen until the measured oxygen concentration matches the target oxygen concentration. In some embodiments, the target oxygen concentration and/or the measured oxygen concentration could be displayed on the device and/or an ancillary device, such as the user's smartphone, smartwatch, or the like. Alternatively, the device could control to the user's blood oxygen saturation (SpO₂) instead of inspired oxygen concentration (FiO₂). This could utilize at least one sensor (such as a pulse oximeter) to measure SpO₂ at a point on the user's body (for example, their finger). This reading could then be displayed somewhere, potentially on the device or the sensor. The user, or the control system could then alter the FiO₂ being delivered using any of the above methods to reach a target SpO₂. In some setups, the FiO₂ or equivalent altitude could be displayed. Additionally, the display could display the altitude equivalent of the current FiO₂. The user may be able to change the target altitude via controls on the display or elsewhere. This target altitude would be converted to a target FiO₂ value by the device controller, which can be used to control the valve. The ability of allowing users to vary the altitude would be desirable for a user who wishes to simulate specific altitudes, instead of targeting specific FiO₂ values.

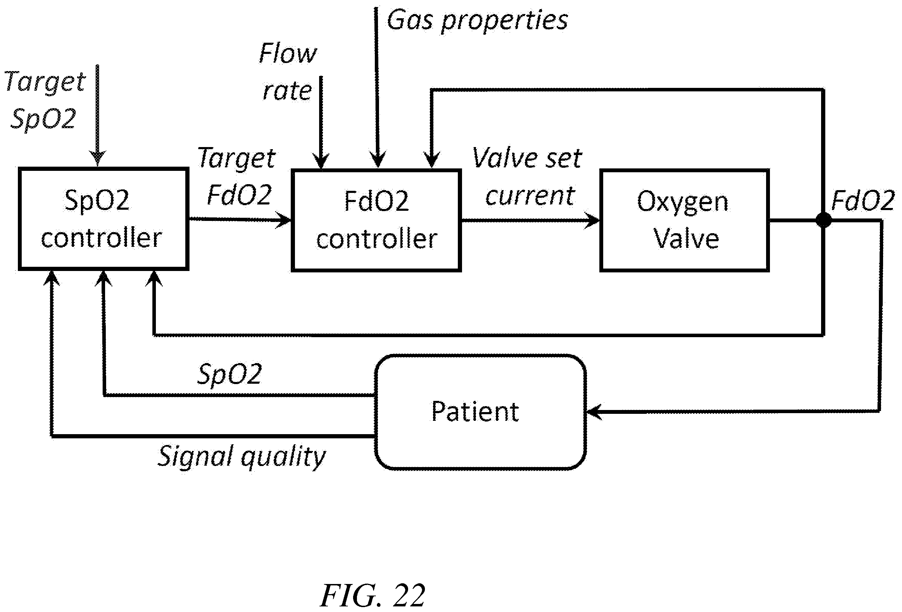

With reference to Figure 22, a schematic diagram of the closed loop control system is illustrated. The closed loop control system may utilize two control loops. The first control loop may be implemented by the SpO₂ controller. The SpO₂ controller can determine a target oxygen concentration, such as FdO₂. Where the gases flow is being delivered as nasal high flow through a non-sealed cannula, assuming the user's inspiratory demand is met, FdO₂ would be substantially equivalent to FiO₂. The determined target oxygen concentration is based at least in part on the target SpO₂ and/or the measured SpO₂. The target SpO₂ value can be a single value or a range of acceptable values. The value(s) could be pre-set, chosen by a user, or determined automatically based on user characteristics. Generally, target SpO₂ values are received or determined before or at the beginning of a therapy session, though target SpO₂ values may be received at any time during the therapy session. During a therapy session, the SpO₂ controller can also receive as inputs: measured FdO₂ reading(s) from a gases composition sensor, and measured SpO₂ reading(s) and a signal quality reading(s) from the physiological sensor. In some configurations, the SpO₂ controller can receive target FdO₂ as an input. In such a case, the output of the SpO₂ controller may be provided directly back to the SpO₂ controller as the input. Based at least in part on the inputs, the SpO₂ controller can output a target FdO₂ to the second control loop.

The second control loop may be implemented by the FdO₂ controller. The FdO₂ controller can receive inputs of measured FdO₂ and target FdO₂. The FdO₂ controller can then output a hypoxic gas inlet valve control signal to control the operation of the hypoxic gas valve based on a difference between these measured FdO₂ and target FdO₂ values. The FdO₂ controller may receive the target FdO₂ value that is output from the first control loop when the flow therapy apparatus is controlling to a target SpO₂. In some configurations, the control signal of the FdO₂ controller may set the current of the hypoxic gas valve in order to control operation of the hypoxic gas valve. Additionally, or alternatively, the FdO₂ controller could detect changes to the measured FdO₂ and alter the position of the valve accordingly. In some configurations, the user manually sets a target SpO₂ or oxygen concentration, and the second control loop can operate independently without receiving the target FdO₂ from the first control loop. Rather, the target FdO₂ can be received from user input or a default value.

The FiO₂ could be controlled within a set of limits, such as pre-determined limits programmed into a memory of device that can be user-customizable in some cases. If the device allows for the supply of nitrogen to be suddenly blocked such that the device returns to delivery of normoxic gas, then such a function could be considered separate and unrestricted by any of the following limits. With each set of limits, an altitude e.g., in meters or feet, can be provided that roughly corresponds to an equivalent partial pressure of oxygen, as this could be displayed alternatively or in addition to the FiO₂ reading. The following FiO₂ limits serve as examples of possible ranges, but the FiO₂ range could be between any two values, including a lower limit from one range and an upper limit from another.
- FiO₂ could be limited between about 20.9*%* (i.e., about 0m) which represents sea level, and about 10.2*%* (i.e., about 5950m altitude) which represents the highest recorded permanently tolerable altitude.
- FiO₂ could be limited or between about 17.4*%* (i.e., about 1600m) which represents approximately the lowest simulated altitude at which the effects of high altitude begin to take effect, and about 11.9*%* (i.e., about 4800m altitude) as lower oxygen concentrations in some cases could require monitoring by a healthcare professional.
- FiO₂ could be limited between about 17.4*%* (i.e., about 1600m) for reasons given above, and about 13.8*%* (i.e., about 3500m altitude) as this is the limit of the "high altitude" region, and higher simulated altitudes may begin to have overly negative effects on training, recovery, sleep quality, appetite, etc. that result in loss of efficacy of the therapy.
- FiO₂ could be limited between about 16.7*%* (i.e., about 2000m) and about 15.7*%* (i.e., about 2500m altitude), which in some cases has been shown to have both benefit and safety advantages.
- FiO₂ could also be limited to other height/altitude equivalents, such as about 500m, about 750m, about 1,000m, about 1,250m, about 1,500m, about 1,750m, about 2,000m, about 2,250m, about 2,500m, about 2,750m, about 3,000m, about 3,250m, about 3,500m, about 3,750m, about 4,000m, or any ranges between the aforementioned values.

In some embodiments, the device could operate based upon a target SpO₂ measurement. One advantage of controlling the hypoxic gas delivery based on SpO₂ is that it allows the device to adjust the FiO₂ (and equivalent altitude) with the exercise intensity. For example, if the user begins exercising at a higher level of intensity, the user's SpO₂ may begin to drop. In response, the device would increase the FiO₂ in order to maintain the target SpO₂. In this situation, the device would effectively be raising the FiO₂ in response to increased exercise intensity. The target SpO₂ could be a pre-set value, a selected value, a pre-set range, a selected range, or a value that changes over time based on a selected therapy program. The SpO₂ reading could be directly fed into the device, and the difference between the target SpO₂ and measured SpO₂ could be used to control a proportional valve on the nitrogen inlet, and therefore the oxygen content of the gas delivered (FiO₂), similar to the one discussed above. As described above, the device controls FiO₂ by changing FdO₂, and assumes inspiratory demand is met, and therefore in such a scenario FiO₂ is equal to FdO₂. If inspiratory demand is not met, the device can still control FdO₂ according to a target SpO₂, as the controller would continue to change FdO₂ (and in turn assumed FiO₂) until the target SpO₂ is achieved. By constantly using feedback from the SpO₂ sensor to control the nitrogen inflow, the target SpO₂ can be reached by altering the oxygen content of the gas. In some embodiments, the measured SpO₂, target SpO₂, and/or FiO₂ can be displayed. The device could include limits on the amount of nitrogen that the valve can allow through and/or a valve position so that an overly dangerous hypoxic composition is not delivered. Additionally, the device could be configured to allow for control of SpO₂ when an SpO₂ sensor is used, but to then default back to one of the earlier FiO₂ control methods when the SpO₂ is not present. Additionally or alternatively, the system can use feedback from other proxy measurements of exertion, such as heart rate, blood pressure, respiratory rate, or heart rate variability to adjust the FiO₂ (and equivalent altitude).

SpO₂ can in some embodiments be controlled between a set of pre-set or other limits. The following are a few non-limiting examples of possible limits, in practice SpO₂ could be limited between any two values, including any reasonable combination of a lower value from one range and an upper value from another.
- SpO₂ could be limited between about 80*%* (the absolute lower limit of what is considered a safe SpO₂ reading in some cases) and about 99*%* or about 100*%* (the upper limit of what is considered a safe SpO₂ reading in some cases).
- SpO₂ could be limited between about 80*%* and about 94*%* (the lower limit of a healthy SpO₂ reading at sea level, and therefore the upper limit of what can be considered altitude training in some cases).
- SpO₂ could be limited between about 85*%* (the lower limit of safe extended exposure without medical supervision in some cases) and about 92*%* (a typical SpO₂ reading at the lower range of high altitude, i.e. about 1600m), in some cases for extended use.
- SpO₂ could be limited between about 87*%* and about 90*%* (a more specific range of SpO₂ that can be both safe and effective in some cases), such as for extended use.
- SpO₂ could be limited between about 80*%* and about 85*%* (the lowest range that can be considered safe in some cases), such as for intermittent use.

The device could be for extended use, for example, all the time (e.g., 24 hours a day); using the device only when awake; using the device only when sleeping; using the device only when exercising; using the device only when not exercising; using the device for a set amount of time, e.g., about, at least about, or no more than about 30 minutes, 45 minutes, 1 hour, 2 hours, 3 hours, 4 hours, 5 hours, 6 hours, 7 hours, 8 hours, 9 hours, 10 hours, 11 hours, 12 hours, 16 hours, 20 hours, or 24 hours a day; or any logical combination of the above (e.g., only when awake and not exercising).

Intermittent use could entail using the device for a set amount of time, such as for example, shorter than about 4, 3, 2, or 1 hour, or using the device for short bursts of about or less than about 10, 9, 8, 7, 6, 5, 4, 3, 2, or 1 minute interspaced with similar short recovery periods of about or less than about 10, 9, 8, 7, 6, 5, 4, 3, 2, or 1 minute, or only using the device while exercising, or any logical combination of the above (e.g., short bursts with interspaced recovery only during exercise).

In some embodiments, the use can be done once a week, or for 2, 3, 4, 5, 6, or 7 days out of a week, 2 weeks, or month for example.

In some embodiments, the use can be continued for a total period of time depending on the desired clinical result. The device can be used over a time period of, for example, about, at least about, or no more than about 1 week, 2 weeks, 3 weeks, 1 month, 2 months, 3 months, 4 months, 5 months, 6 months, 9 months, 12 months, or more or less.

The device could be used in a therapy program that contains treatment periods that could be considered extended use as well as other periods that could be considered intermittent use, and could use different SpO₂ target ranges appropriate for both. For example, the device could be used in a lower SpO₂ target range while exercising, as well as a higher SpO₂ target range when not exercising for the rest of the day.

In some embodiments, personalized training methods and altitude equivalent oxygen concentrations for therapy can determined by observing how different individuals acclimatize at different rates. In general, less than about 1250 meters above sea level is commonly described as "low altitude", as noticeable effects generally begin to take place at about 1500 meters. From about 1500 meters to about 3500 meters is often referred to as the "high altitude" region, with regions over about 3500 meters being the "very high altitude", and can be potentially dangerous to train at in some cases, especially if not given the proper time to acclimatize. High altitude training tends to occur in the region about 2000 meters to about 2500 meters, although this varies with training regimes, particularly the activity performed in the high altitude region and the length of time spent there. Some activity regimes include:
- Live high/train high: This is likely the simplest form of altitude training, as it simply consists of remaining at altitude (e.g., breathing an altitude-equivalent oxygen concentration of the hypoxic gas composition) for the entire length of the therapy, including at training times.
- Live high/train low: This involves exposing oneself to altitude (e.g. >2000m meters) (e.g., breathing an altitude-equivalent oxygen concentration of the hypoxic gas composition) during non-training periods, but returning to a low altitude environment (i.e., less than about 1250 meters) (e.g., breathing an altitude-equivalent oxygen concentration of the hypoxic gas composition) for exercise. In some embodiments, the low altitude environment can be achieved by utilizing systems and methods as disclosed herein with supplemental oxygen during non-training periods (e.g., non-hypoxic gas compositions with oxygen concentrations of at least about 21*%*, 22*%*, 23*%*, 24*%*, 25*%*, 30*%*, 35*%*, 40*%*, 45*%*, 50*%*, or more, or ranges encompassing two or more of the aforementioned values). This is thought to allow for the benefits of acclimatization while still maintaining the same exercise intensity. A study using these altitude ranges showed improvements in speed, strength, endurance and recovery.
- Live low/train high: This involves athletes training at high altitude (e.g., breathing an altitude-equivalent oxygen concentration of the hypoxic gas composition) and spending the remaining non-training periods in a low oxygen environment. The idea is increase the strain on the user's cardiovascular system while allowing them to recover in a normoxic environment. This has the added benefit of reducing the time needed to be spent in hypoxia.
- Repeated sprints in hypoxia: This involves the athlete performing a high intensity exercise (e.g., sprinting) for a short period followed by a rest period, and then repeating the cycle, all in hypoxic conditions. In some embodiments, the high intensity exercise could be performed for, for example, between about 15 seconds and about 180 seconds, such as about 15, 30, 60, 75, 90, 105, 120, 150, or 180 seconds, or ranges incorporating any two of the aforementioned values. In some embodiments, the rest period can be, for example, about, at least about, or no more than about 1x, 1.5x, 2x, 2.5x, 3x, 3.5x, 4x, 4.5x, 5x the exercise period or ranges incorporating any two of the aforementioned values. As one non-limiting example, high intensity exercise is performed for about 30 seconds, and then a rest period for about or no more than about 120 seconds, followed by additional cycles of high intensity exercise and rest. In some embodiments, the user can perform between about 2 cycles and about 20 cycles under this protocol, such as between about 2 cycles and about 12 cycles, between about 5 cycles and about 10 cycles, or about 2, 3, 4, 5, 6, 7, 8, 9, 10, 11, 12, 13, 14, 15, 16, 17, 18, 19, or 20 cycles, or ranges incorporating any two of the aforementioned values. While this can be seen as a variation on live low/train high, the underlying mechanisms are theorized to achieve different results. This was tested on athletes who were able to complete 9-10 sprints before total exhaustion. The athletes were split into two groups that performed the above exercises, however one group did it in hypoxia and the other in normoxia. After 4 weeks the athletes who performed the sprints in hypoxia could manage 13 before total exhaustion, while the normoxic group had shown no improvement.
- In some configurations, an exercise program can include varying FiO₂ (or FdO₂) by increasing or decreasing FiO₂ (or FdO₂) during a training session based on a pre-set sequence. For example, the FiO₂ could start at or near 21*%* and slowly drop to a specific hypoxic value, and then slowly ramp back up to around 21*%* near the end of the session, or vice versa. The varying of FiO₂ (or FdO₂) can be performed gradually, in a series of step changes, and/or in one step change. The varying of FiO₂ (or FdO₂) can be repeated during the training session. This could be done to simulate rising from sea level to high altitude, followed by descending back to sea level. The FiO₂ could also be varied based on other determined exercise regimes. For example, an exercise session could involve high and low intensity phases, with the FiO₂ programmed to coordinate with these phases. This could involve lower FiO₂ values being set for periods of low exercise intensity, with the FiO₂ being raised for periods of high intensity. The device could also assess exercise intensity level and automatically adjust the FiO₂ based on the determined exercise intensity level. Assessing intensity level could be done by monitoring one or more physiological parameters, such as heart rate, respiratory rate, blood pressure, heart rate variability, and/or the like. When performing the pre-set exercise programs, the user's SpO₂ could be monitored (e.g., by a pulse oximeter or otherwise) to ensure that the user's SpO₂ does not drop below a predetermined value. When the user's SpO₂ drops below a predetermined value, the device or system can manually override the target FiO₂ value or target altitude equivalent value.

### Controller

Figure 19B illustrates a block diagram of an embodiment of a controller 600. The controller 600 can include programming instructions for detection of input conditions and control of output conditions. The programming instructions can be stored in a memory 624 of the controller 600. The programming instructions can correspond to the methods, processes and functions described herein. The programming instructions can be executed by one or more hardware processors 622 of the controller 600. The programming instructions can be implemented in C, C++, JAVA, or any other suitable programming languages. Some or all of the portions of the programming instructions can be implemented in application specific circuitry 628 such as ASICs and FPGAs.

The controller 600 can also include circuits 628 for receiving sensor signals. The controller 600 can further include a display 630 for transmitting status of the user and the respiratory assistance system. The display 630 can also show warnings. The display 630 can be configured to display characteristics of sensed gas(es) in real time. The controller 600 can also receive user inputs via the user interface such as display 630. The user interface may alternatively or additionally comprise buttons or a dial. The user interface may alternatively or additionally comprise a touch screen.

### Motor/Sensor module

Any of the features of the flow therapy apparatus described herein, including but not limited to the humidifier, the flow generator, the user interface, the controller, and the user breathing conduit configured to couple the gases flow outlet of the respiratory system to the user interface, can be combined with any of the sensor modules described herein.

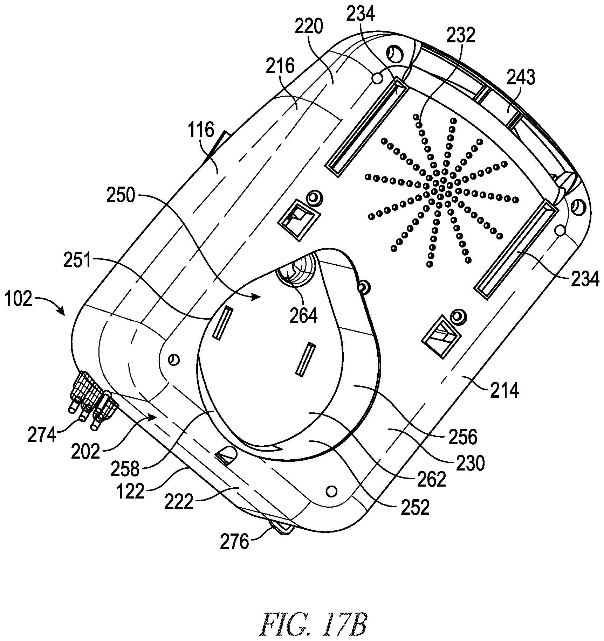

Figure 20 illustrates a block diagram of the motor/sensor module 2000, which is received by the recess 250 in the flow therapy apparatus (shown in Figures 17A and 17B). The motor/sensor module can include a blower 2001, which entrains room air to deliver to a user. The blower 2001 can be a centrifugal blower.

Room air can enter an ambient/room air inlet 2002, which enters the blower 2001 through an inlet port 2003. The inlet port 2003 can include a valve 2004 through which a pressurized gas may enter the blower 2001. The valve 2004 can control a flow of nitrogen or other auxiliary gas into the blower 2001. The valve 2004 can be any type of valve, including a proportional valve or a binary valve. In some embodiments, the valve can be on the nitrogen supply line, e.g., a nitrogen source bottle or other reservoir, and manually or automatically adjusted to alter the amount of nitrogen being delivered. In some embodiments, the inlet port does not include a valve.

The blower 2001 can operate at a motor speed of greater than 1,000 RPM (revolutions per minute) and less than 30,000 RPM, greater than 2,000 RPM and less than 21,000 RPM, or between any of the foregoing values. Operation of the blower 2001 mixes the gases entering the blower 2001 through the inlet port 2003. Using the blower 2001 as the mixer can decrease the pressure drop that would otherwise occur in a system with a separate mixer, such as a static mixer comprising baffles, because mixing requires energy.

The mixed air can exit the blower 2001 through a conduit 2005 and enters the flow path 2006 in the sensor chamber 2007. A sensing circuit board with sensors 2008 can positioned in the sensor chamber 2007 such that the sensing circuit board is at least partially immersed in the gases flow. At least some of the sensors 2008 on the sensing circuit board can be positioned within the gases flow to measure gas properties within the flow. After passing through the flow path 2006 in the sensor chamber 2007, the gases can exit 2009 to the humidification chamber.

Positioning sensors 2008 downstream of the combined blower and mixer 2001 can increase accuracy of measurements, such as the measurement of gases fraction concentration, including nitrogen and/or oxygen concentration, over systems that position the sensors upstream of the blower and/or the mixer. Such a positioning can give a repeatable flow profile. Further, positioning the sensors downstream of the combined blower and mixer avoids the pressure drop that would otherwise occur, as where sensing occurs prior to the blower, a separate mixer, such as a static mixer with baffles, is required between the inlet and the sensing system. The mixer can introduce a pressure drop across the mixer. Positioning the sensing after the blower can allow the blower to be a mixer, and while a static mixer would lower pressure, in contrast, a blower increases pressure. Also, immersing at least part of the sensing circuit board and sensors 2008 in the flow path can increase the accuracy of measurements because the sensors being immersed in the flow means they are more likely to be user to the same conditions, such as temperature and pressure, as the gases flow and therefore provide a better representation of the gas characteristics.

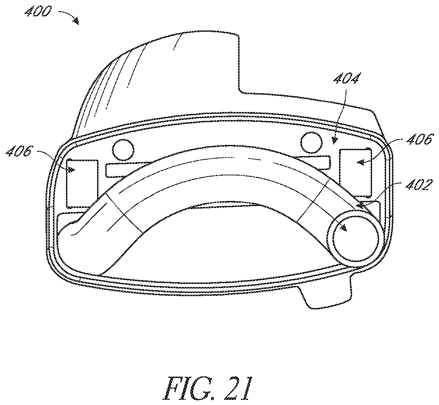

Turning to Figure 21, the gases exiting the blower can enter a flow path 402 in the sensor chamber 400, which can be positioned within the motor and/or sensor module. The flow path 402 can have a curved shape. The flow path 402 can be configured to have a curved shape with no sharp turns. The flow path 402 can have curved ends with a straighter section between the curved ends. A curved flow path shape can reduce pressure drop in a gases flow without reducing the sensitivity of flow measurements by partially coinciding a measuring region with the flow path to form a measurement portion of the flow path.

A sensing circuit board 404 with sensors, such as ultrasonic transmitters, receivers, humidity sensor, temperature sensor, flow rate sensor, and the like, can be positioned in the sensor chamber 400 such that the sensing circuit board 404 is at least partially immersed in the flow path 402. Immersing at least part of the sensing circuit board and sensors in the flow path can increase the accuracy of measurements because the sensors immersed in the flow are more likely to be user to the same conditions, such as temperature and pressure, as the gases flow, and therefore provide a better representation of the characteristics of the gases flow. After passing through the flow path 402 in the sensor chamber 400, the gases can exit to the humidification chamber.

With continued reference to Figure 21, openings 406 of the sensor chamber 400 can hold acoustic transmitters, such as ultrasonic transducers which form an acoustic axis along at least a portion of the flow path 402 to measure properties or characteristics of the gases within the flow. The ultrasonic transducers can act as both transmitters and receivers.

### Example 1

A human user performed a 3 km trial run on a gravel track and recorded a time of 10 minutes and 54 seconds. The user's best time over recent years has been 10 minutes and 38 seconds. 2 weeks later, the user commenced use of a high flow device, such as a high flow device coupled to a nasal cannula patient interface as disclosed herein with delivery of a hypoxic gas composition including enriched nitrogen at an altitude equivalent of about 2,000 m (about 16.7% oxygen concentration in the hypoxic gas composition) for 2-3 hours a night, 5 days a week, for a total of three weeks. During this time, the user typically maintained the hypoxic gas flow rate at between about 5 liters/minute and about 8 liters/minute, and occasionally raised the hypoxic gas flow rate to at least 15 liters/minute for a couple minutes to verify its effects on his blood saturation, and using a pulse oximeter was able to observe his blood oxygen saturation dropping below 90%. After three weeks of the therapy, the user took a week off with no therapy, and then ran a 3km trial run on a grass track (which typically produces much slower times than gravel) and recorded a much faster time of 10 minutes and 15 seconds. The user attributed the surprising and unexpected improvement in his run time to use of the high flow device disclosed herein.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Furthermore, certain features that are described in this disclosure in the context of separate implementations can also be implemented in combination in a single implementation. Conversely, various features that are described in the context of a single implementation can also be implemented in multiple implementations separately or in any suitable subcombination.

Moreover, while operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. Furthermore, the features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount.

The scope of the present invention is not intended to be limited by the specific disclosures of embodiments in this section or elsewhere in this specification, and instead is defined by the appended claims in this specification. The language of the claims is to be interpreted broadly based on the language employed in the claims and not limited to the examples described in the present specification or during the prosecution of the application, which examples are to be construed as non-exclusive.

## Claims

1. A nasal high flow system (10) configured for providing a hypoxic flow of gases to a user, comprising:
an apparatus comprising a gas flow path, an ambient air inlet (356'), and a hypoxic gas source inlet (358') configured to connect to a hypoxic gas source and configured to create a hypoxic gas composition upon mixing of ambient air and hypoxic gas;
a user interface (17) comprising a nasal cannula comprising nasal prongs (18);
a gas flow generation element (11) configured to deliver the hypoxic gas composition to nares of the user at a predetermined high flow rate of at least about 10 liters/minute;
a sensor (99) configured to measure a physiological parameter of the user, wherein the physiological parameter is blood oxygen saturation; and
a controller (13) configured to automatically control the delivery of the hypoxic gas composition based on the measured physiological parameter;
wherein the nasal cannula is non-sealed.

2. The system (10) of Claim 1, wherein the gas flow generation element (11) comprises one or more of a blower, a compressor, a pressurized tank, or a gas source in the wall.

3. The system (10) of any of Claims 1-2, wherein the hypoxic gas comprises enriched nitrogen.

4. The system (10) of any of Claims 1-3, wherein the gas flow generation element (11) is configured to deliver the hypoxic gas composition sufficient to meet the peak inspiratory demand of the user.

5. The system (10) of any of Claims 1-4, further comprising a heating element (16a) configured to heat the hypoxic gas composition prior to reaching the nares of the user.

6. The system (10) of any of Claims 1-5, further comprising a humidification element (12) configured to humidify the hypoxic gas composition prior to reaching the nares of the user.

7. The system (10) of any of Claims 1-6, further comprising an alarm configured to notify the user when the physiological parameter deviates from a pre-selected range for at least a pre-determined period of time.

8. The system (10) of Claim 7, wherein the alarm comprises one of a visual, auditory, or tactile alarm.

9. The system (10) of any of Claims 1-8, wherein the controller (13) is configured to titrate the composition of the hypoxic gas composition to achieve a blood oxygen saturation of the user of less than about 95%.

10. The system (10) of any of Claims 1-9, wherein the controller (13) is configured to automatically control the delivery of the hypoxic gas composition by adjusting an amount of hypoxic gas mixed into the hypoxic gas composition.

11. The system (10) of any of Claims 1-10, further comprising a sensor configured to sense the oxygen concentration of the hypoxic gas composition.

12. The system (10) of Claim 11, wherein the system (10) is further configured to obtain a target oxygen concentration of the hypoxic gas composition based on the blood oxygen saturation.

13. The system (10) of any of Claims 1-12, wherein the nasal high flow system (10) is a high flow device.

14. The system (10) of Claim 13, wherein the high flow device is portable.

## Patentansprüche

1. Nasales Hochfluss-System (10), das zum Bereitstellen eines hypoxischen Gasflusses für einen Benutzer konfiguriert ist, umfassend:
eine Einrichtung, die einen Gasflusspfad, einen Umgebungslufteinlass (356') und einen Einlass (358') für eine hypoxische Gasquelle umfasst, der konfiguriert ist, um mit einer hypoxischen Gasquelle verbunden zu werden, und konfiguriert ist, um beim Mischen von Umgebungsluft und hypoxischem Gas eine hypoxische Gaszusammensetzung zu erzeugen;
eine Benutzerschnittstelle (17), die eine Nasenkanüle umfasst, die eine Nasenklammer (18) umfasst;
ein Gasflusserzeugungselement (11), das konfiguriert ist, um die hypoxische Gaszusammensetzung mit einer vorbestimmten hohen Flussrate von mindestens etwa 10 Litern/Minute in die Nasenlöcher des Benutzers abzugeben;
einen Sensor (99), der konfiguriert ist, um einen physiologischen Parameter des Benutzers zu messen, wobei der physiologische Parameter die Blutsauerstoffsättigung ist; und
eine Steuereinheit (13), die konfiguriert ist, um automatisch die Abgabe der hypoxischen Gaszusammensetzung basierend auf dem gemessenen physiologischen Parameter zu steuern;
wobei die Nasenkanüle nicht versiegelt ist.

2. System (10) nach Anspruch 1, wobei das Gasflusserzeugungselement (11) eines oder mehr von einem Gebläse, einem Kompressor, einem Druckbehälter oder einer Gasquelle in der Wand umfasst.

3. System (10) nach einem der Ansprüche 1-2, wobei das hypoxische Gas angereicherten Stickstoff umfasst.

4. System (10) nach einem der Ansprüche 1-3, wobei das Gasflusserzeugungselement (11) konfiguriert ist, um die hypoxische Gaszusammensetzung ausreichend abzugeben, um den Spitzen-Einatmungsbedarf des Benutzers zu decken.

5. System (10) nach einem der Ansprüche 1-4, das weiter ein Heizelement (16a) umfasst, das konfiguriert ist, um die hypoxische Gaszusammensetzung vor Erreichen der Nasenlöcher des Benutzers zu erwärmen.

6. System (10) nach einem der Ansprüche 1-5, das weiter ein Befeuchtungselement (12) umfasst, das konfiguriert ist, um die hypoxische Gaszusammensetzung vor Erreichen der Nasenlöcher des Benutzers zu befeuchten.

7. System (10) nach einem der Ansprüche 1-6, das weiter einen Alarm umfasst, der konfiguriert ist, um den Benutzer zu benachrichtigen, wenn der physiologische Parameter mindestens eine vorbestimmte Zeit lang von einem vorausgewählten Bereich abweicht.

8. System (10) nach Anspruch 7, wobei der Alarm eines von einem visuellen, akustischen oder taktilen Alarm umfasst.

9. System (10) nach einem der Ansprüche 1-8, wobei die Steuereinheit (13) konfiguriert ist, um die Zusammensetzung der hypoxischen Gaszusammensetzung zu titrieren, um eine Blutsauerstoffsättigung des Benutzers von weniger als etwa 95% zu erreichen.

10. System (10) nach einem der Ansprüche 1-9, wobei die Steuereinheit (13) konfiguriert ist, um automatisch die Abgabe der hypoxischen Gaszusammensetzung durch Anpassen einer Menge an hypoxischem Gas, die in die hypoxische Gaszusammensetzung gemischt wird, zu steuern.

11. System (10) nach einem der Ansprüche 1-10, das weiter einen Sensor umfasst, der konfiguriert ist, um die Sauerstoffkonzentration der hypoxischen Gaszusammensetzung zu erfassen.

12. System (10) nach Anspruch 11, wobei das System (10) weiter konfiguriert ist, um eine Zielsauerstoffkonzentration der hypoxischen Gaszusammensetzung basierend auf der Blutsauerstoffsättigung zu erhalten.

13. System (10) nach einem der Ansprüche 1-12, wobei das nasale Hochfluss-System (10) eine Hochflussvorrichtung ist.

14. System (10) nach Anspruch 13, wobei die Hochflussvorrichtung tragbar ist.

## Revendications

1. Système nasal à haut débit (10) configuré pour fournir un flux hypoxique de gaz à un utilisateur, comprenant :
un appareil comprenant un trajet d'écoulement de gaz, une entrée d'air ambiant (356') et une entrée de source de gaz hypoxique (358') configurée pour être raccordée à une source de gaz hypoxique et configurée pour créer une composition de gaz hypoxique lors du mélange d'air ambiant et de gaz hypoxique ;
une interface utilisateur (17) comprenant une canule nasale comprenant des lunettes nasales (18) ;
un élément de génération de flux de gaz (11) configuré pour distribuer la composition de gaz hypoxique aux narines de l'utilisateur à un débit élevé prédéterminé d'au moins environ 10 litres/minute ;
un capteur (99) configuré pour mesurer un paramètre physiologique de l'utilisateur, dans lequel le paramètre physiologique est la saturation en oxygène du sang ; et
un dispositif de commande (13) configuré pour commander automatiquement la distribution de la composition de gaz hypoxique sur la base du paramètre physiologique mesuré ;
dans lequel la canule nasale n'est pas scellée.

2. Système (10) selon la revendication 1, dans lequel l'élément de génération de flux de gaz (11) comprend un ou plusieurs éléments parmi une soufflante, un compresseur, un réservoir sous pression ou une source de gaz dans la paroi.

3. Système (10) selon l'une quelconque des revendications 1-2, dans lequel le gaz hypoxique comprend de l'azote enrichi.

4. Système (10) selon l'une quelconque des revendications 1-3, dans lequel l'élément de génération de flux de gaz (11) est configuré pour distribuer la composition de gaz hypoxique suffisante pour répondre à la demande inspiratoire de pointe de l'utilisateur.

5. Système (10) selon l'une quelconque des revendications 1-4, comprenant en outre un élément chauffant (16a) configuré pour chauffer la composition de gaz hypoxique avant d'atteindre les narines de l'utilisateur.

6. Système (10) selon l'une quelconque des revendications 1-5, comprenant en outre un élément d'humidification (12) configuré pour humidifier la composition de gaz hypoxique avant d'atteindre les narines de l'utilisateur.

7. Système (10) selon l'une quelconque des revendications 1-6, comprenant en outre une alarme configurée pour avertir l'utilisateur lorsque le paramètre physiologique s'écarte d'une plage présélectionnée pendant au moins une période de temps prédéterminée.

8. Système (10) selon la revendication 7, dans lequel l'alarme comprend l'une parmi une alarme visuelle, auditive ou tactile.

9. Système (10) selon l'une quelconque des revendications 1-8, dans lequel le dispositif de commande (13) est configuré pour titrer la composition de la composition de gaz hypoxique pour obtenir une saturation en oxygène du sang de l'utilisateur inférieure à environ 95 %.

10. Système (10) selon l'une quelconque des revendications 1-9, dans lequel le dispositif de commande (13) est configuré pour commander automatiquement la distribution de la composition de gaz hypoxique en ajustant une quantité de gaz hypoxique mélangée dans la composition de gaz hypoxique.

11. Système (10) selon l'une quelconque des revendications 1-10, comprenant en outre un capteur configuré pour détecter la concentration en oxygène de la composition de gaz hypoxique.

12. Système (10) selon la revendication 11, dans lequel le système (10) est en outre configuré pour obtenir une concentration cible en oxygène de la composition de gaz hypoxique sur la base de la saturation en oxygène du sang.

13. Système (10) selon l'une quelconque des revendications 1-12, dans lequel le système nasal à haut débit (10) est un dispositif à haut débit.

14. Système (10) selon la revendication 13, dans lequel le dispositif à haut débit est portable.
